# FASCICULE DE BREVET EUROPEEN

(11) **EP 2 462 219 B1**
(45) Date de publication et mention de la délivrance du brevet: **24.06.2015**
(21) Numéro de dépôt: 10740205.9
(22) Date de dépôt: 30.07.2010
(51) Int. Cl.: C12M 1/22

(54) **DISPOSITIF ET PROCÉDÉ DE DISTRIBUTION D'UN PRODUIT DANS UNE BOÎTE DE PETRI**
VORRICHTUNG UND VERFAHREN AUSGABE EINES MATERIALS IN EINE PETRISCHALE
DEVICE AND METHOD FOR DISPENSING A MATERIAL INTO A PETRI DISH

(30) Priorité: 07.08.2009 FR 0955568
(43) Date de publication de la demande: 13.06.2012
(73) Titulaire: AES Chemunex, 35270 Combourg (FR)
(72) Inventeur: BRELIVET, Nicolas, F-22350 Saint Jouan De L'isle (FR)
(74) Mandataire: Regimbeau
(86) Numéro de dépôt international: PCT/EP2010/061104
(87) Numéro de publication internationale: WO 2011/015528

(56) Documents cités:
- EP-A1- 0 385 902
- WO-A2-97/00002
- GB-A- 2 018 288
- JP-A- 3 049 676
- US-B1- 6 199 605
- DATABASE WPI Week 200377 Thomson Scientific, London, GB; AN 2003-820093 XP002579637, & JP 2003 225083 A (SONY CORP) 12 août 2003 (2003-08-12)

## Description

L'invention concerne un dispositif de distribution d'un produit prescrit, notamment de gélose, dans des boîtes de Petri.

Les boîtes de Petri sont de petites boîtes de contour circulaire, en matière plastique transparente, qui contiennent un produit nutritif tel qu'un milieu gélosé ; elles servent de milieu de développement pour des cultures contenant des micro-organismes et sont utilisées en grand nombre par les laboratoires d'analyse, notamment dans la recherche médicale et l'industrie.

Chaque boîte de Petri comporte un couvercle amovible apte à être disposé sur un fond moins large que celui-ci.

Un dispositif selon le préambule de la revendication 1 est connu par le document JP-A-03 049 676.

Pour distribuer du produit, un dispositif mécanique est prévu pour ouvrir la boîte et amener le fond ouvert de celle-ci à un poste de distribution de produit par une tête de distribution comportant un embout de sortie du produit vers le fond.

L'un des problèmes posé par ce poste de distribution tient à la consistance non liquide du produit qui y est distribué.

En effet, le plus souvent ce produit est visqueux, notamment dans le cas de la gélose.

Du fait de sa viscosité, le produit tend à ne pas être réparti uniformément dans le fond de boîte de Petri.

Ce problème s'aggrave encore dans le cas où l'on distribue une petite quantité dans le fond de la boîte de Petri.

On cherche donc à obtenir un dispositif de distribution pour répartir uniformément le produit dans le fond de la boîte de Petri, et ce quelle que soit la quantité de produit distribué et sans risque de répandre du produit hors du fond de la boîte de Petri.

A cet effet, un premier objet de l'invention est un dispositif de distribution d'un produit prescrit dans au moins une boîte de Petri; chaque boîte de Petri comportant un couvercle amovible apte à être disposé sur un fond moins large que celui-ci,
le dispositif comportant des moyens d'amenée de la boîte de Petri à un poste de distribution, où la boîte de Petri est maintenue avec un espace entre son couvercle et son fond pour permettre la distribution du produit par une tête de distribution du produit comportant un embout de sortie de produit vers le fond lorsque l'embout est dans une position de distribution,
caractérisé en ce que le dispositif comporte en outre un piston comportant un plateau de support du fond au poste de distribution du produit et des moyens d'entraînement en rotation du plateau sur lui-même par rapport à la tête de distribution, agencés pour faire tourner le fond par rapport à la tête de distribution se trouvant dans la position de distribution du produit dans le fond, dans laquelle l'embout de sortie de produit est agencé par rapport au plateau pour distribuer du produit dans le fond mis en rotation,
les moyens d'amenée comportant un organe de transfert du fond jusqu'à une position d'arrêt déterminée au poste de distribution, l'organe de transfert ayant une ouverture supérieure de support du couvercle de la boîte de Petri au-dessus d'une ouverture inférieure de support du fond de la boîte de Petri, ledit plateau déterminé étant apte à traverser les ouvertures pour faire remonter le fond de la boîte de Petri dans le couvercle en traversant leur ouverture respective de support, lorsque l'organe de transfert se trouve dans la position d'arrêt déterminée au poste de distribution,
le piston déterminé muni du plateau déterminé ayant la fonction de faire tourner le fond de la boîte de Petri pendant la distribution du produit dans le fond dans ladite position de distribution et ayant la fonction de soulever le fond de la boîte de Petri au travers de l'ouverture supérieure pour rentrer le fond dans le couvercle de la boîte de Petri dans ladite position d'arrêt déterminée.

Suivant des modes de réalisation de l'invention :
- La vitesse de rotation du plateau sur lui-même est comprise entre 10 et 100 tours par minute.
- Les moyens d'entraînement en rotation sont prévus pour faire tourner le plateau sur lui-même autour d'un axe géométrique de rotation, l'embout comporte une extrémité de sortie de produit située à distance de l'axe géométrique de rotation sur lui-même du plateau mis en rotation dans la position de distribution.
- Les moyens d'entraînement en rotation sont prévus pour faire tourner le plateau sur lui-même autour d'un axe géométrique de rotation, l'embout de sortie est agencé pour déverser le produit en une zone du fond, laquelle zone est située à distance de l'axe géométrique de rotation du plateau mis en rotation dans la position de distribution.
- Le plateau comporte une surface supérieure concave de son centre vers sa périphérie pour la réception du fond de la boîte de Petri.
- Ladite surface supérieure du plateau comporte une partie de contact périphérique avec le fond de la boîte de Petri.
- Le plateau comporte un évidement en son centre sur sa surface supérieure.
- La surface supérieure du plateau comporte une première partie périphérique de contact avec le fond de la boîte de Petri, laquelle première partie de contact est en un matériau présentant une plus grande adhérence que le matériau d'une deuxième partie de la surface supérieure du plateau, entourée par cette première partie périphérique de contact.
- La première partie périphérique de contact est un matériau caoutchouteux, tandis que la deuxième partie entourée par cette première partie périphérique de contact est métallique.
- Une colonne de guidage vertical de boîtes de Petri est disposée au-dessus du piston pour recevoir la boîte de Petri ayant son fond remonté dans son couvercle par ledit piston au poste de remplissage, le piston étant agencé pour déplacer la boîte de Petri ayant son fond remonté dans son couvercle de l'organe de transfert à ladite colonne de guidage vertical de boîtes de Petri, ladite colonne de guidage vertical de boîtes de Petri comportant des moyens de retenue de la boîte de Petri ayant son fond remonté dans son couvercle ayant été amenée par le piston.

Un autre objet de l'invention est un procédé de distribution d'un produit prescrit dans au moins une boite de Petri à l'aide du dispositif de distribution tel que décrit ci-dessus, chaque boîte de Petri comportant un couvercle amovible apte à être disposé sur un fond moins large que celui-ci, procédé dans lequel, on ouvre la boîte de Petri pour maintenir son couvercle à distance de son fond, on amène le fond à un poste de distribution, on met la tête de distribution dans une position de distribution pour envoyer du produit dans le fond se trouvant au poste de distribution, caractérisé en ce que l'on positionne le fond de la boîte de Petri sur un piston comportant un plateau tournant sur lui-même pour faire tourner le fond sur lui-même pendant toute la durée de distribution du produit dans le fond depuis la tête de distribution en position de distribution audit poste,
le fond est amené jusqu'à une position d'arrêt déterminée au poste de distribution par un organe de transfert ayant une ouverture supérieure de support du couvercle de la boîte de Petri au-dessus d'une ouverture inférieure de support du fond de la boîte de Petri, ledit plateau déterminé étant apte à traverser les ouvertures pour faire remonter le fond de la boîte de Petri dans le couvercle en traversant leur ouverture respective de support, lorsque l'organe de transfert se trouve dans la position d'arrêt déterminée au poste de distribution,
le piston déterminé muni du plateau déterminé faisant tourner le fond de la boîte de Petri pendant la distribution du produit dans le fond dans ladite position de distribution, puis soulevant le fond de la boîte de Petri au travers de l'ouverture supérieure pour rentrer le fond dans le couvercle de la boîte de Petri dans ladite position d'arrêt déterminée au poste de distribution.

L'invention sera mieux comprise à la lecture de la description qui va suivre, donnée uniquement à titre d'exemple non limitatif en référence aux dessins annexés, sur lesquels :
- la figure 1 est une vue schématique d'un dispositif de distribution muni d'un poste de remplissage de produit suivant l'invention,
- la figure 2A est une vue schématique en perspective d'un plateau de piston utilisé dans le dispositif suivant la figure 1,
- la figure 2B est une vue schématique en coupe transversale du plateau suivant la figure 2A,
- la figure 3 est une vue schématique de côté du piston dans une position abaissée sous le fond d'une boîte de Petri dans un mode de réalisation du dispositif suivant l'invention,
- la figure 4 est une vue schématique de côté du piston dans une position d'élévation du fond d'une boîte de Petri pour la distribution de produit dans un mode de réalisation du dispositif suivant l'invention,
- la figure 5 est une vue schématique partielle en perspective du poste de distribution dans une position d'escamotage, correspondant à la figuré 9B, dans un mode de réalisation du dispositif suivant l'invention,
- la figure 6A est une vue schématique partielle en perspective du poste de distribution dans une position de distribution, correspondant aux figures 1, 4 et 9C, dans un mode de réalisation du dispositif suivant l'invention,
- la figure 6B est une vue schématique partielle de dessus du poste de distribution selon la figure 6A dans un mode de réalisation du dispositif suivant l'invention,
- la figure 7 est une vue schématique en perspective d'un mode de réalisation d'une machine mettant en oeuvre le dispositif de distribution suivant l'invention,
- la figure 8 est une vue schématique en perspective d'une partie du dispositif de la figure 7,
- les figures 9A, 9B, 9C, 9D, 9E, 9F et 9G sont des vues partielles agrandies du dispositif suivant la figure 7 dans différentes positions successives de fonctionnement pour le remplissage d'une boîte de Petri, et
- les figures 10A, 10B, 10C et 10D sont des vues schématiques de profil d'une partie du dispositif suivant les figures précédentes dans des positions différentes.

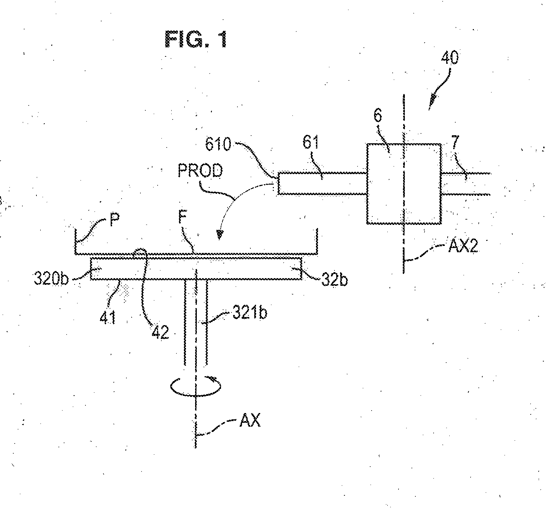

A la figure 1, un poste 40 de distribution de produit dans un fond F d'une boîte P de Petri est représenté. Ce produit est par exemple de la gélose. Ainsi que représenté aux autres figures, chaque boîte P de Petri comporte un fond F et un couvercle C. Le poste 40 comporte une tête 6 de distribution de produit ayant un embout 61 de sortie de produit vers le fond F.

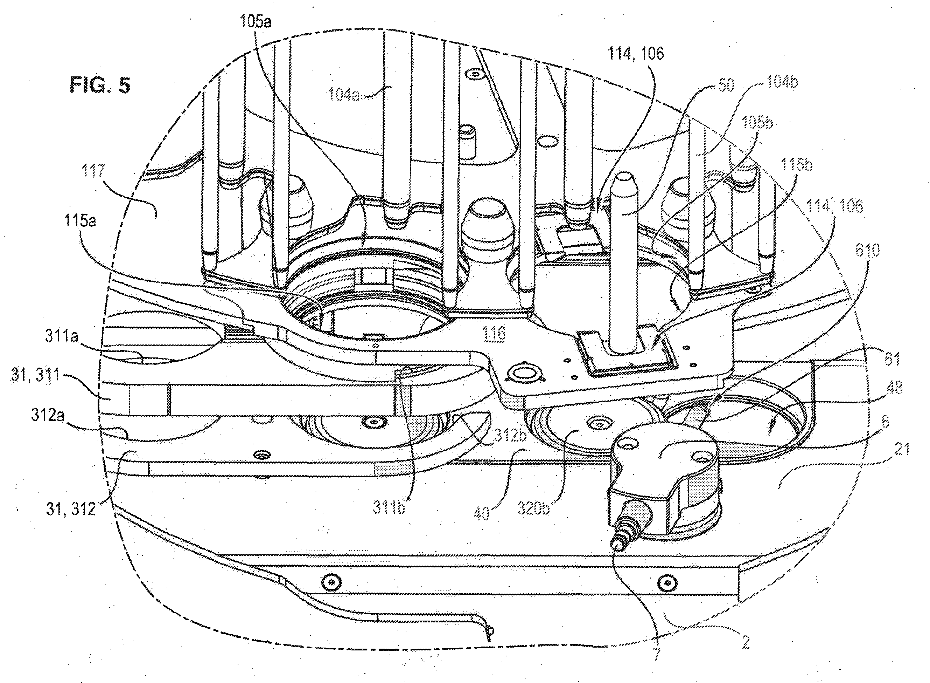

Le poste 40 de distribution comporte également un piston 32b comportant un plateau supérieur 320b pour la réception du fond F d'une boite de Petri. Le fond F de la boîte de Petri est amené par des moyens appropriés sur le plateau 320b pour y déverser du produit par la tête 6. Ces moyens de déplacement du fond F de la boîte de Pétri jusqu'au poste 40 de distribution sont par exemple ceux du mode de réalisation décrit ci-dessous en référence aux figures 5 et suivantes. Ces moyens permettent d'ouvrir la boîte P de Pétri pour qu'il existe un espace entre son fond F et son couvercle C. Ainsi, au poste 40 de distribution, la tête 6 peut être placée dans une position de distribution dans laquelle l'embout 61 a son extrémité 610 de sortie située au-dessus du fond F ouvert situé sur le plateau 320b.

Le plateau 320b est relié à des moyens d'entrainement en rotation du plateau 320b sur lui-même. Par exemple, le plateau 320b est relié à son côté inférieur 41 à une broche centrale 321b fixée solidairement au plateau 320b. En faisant tourner sur elle-même la broche centrale 321b, on fait tourner sur lui-même le plateau 320b. Un moteur électrique permet d'entraîner par une courroie et un engrenage la broche centrale 321b et donc le plateau 320b sur lui-même. Le plateau 320b a une forme générale plane, par exemple horizontale, et la rotation sur lui-même du plateau 320b s'effectue dans ce plan horizontal.

Des moyens de commande de la tête 6 sont prévus pour envoyer du produit dans l'embout 61 en position de distribution lorsque le plateau 320b tourne. La rotation du plateau sur lui-même permet de répartir le produit déversé par l'embout 61 sur toute la surface du fond F de la boîte de Petri en évitant de répandre du produit en dehors du fond F.

Ainsi, le fond F est maintenu en rotation sur lui-même pendant toute la durée de déversement de produit depuis l'embout 61 en position de distribution.

On rend ainsi plus uniforme la répartition spatiale du produit dans le fond F de la boîte de Petri, en minimisant l'encombrement, en évitant d'agiter le fond F et en distribuant simultanément le produit par la tête 6 dans le fond F, pour un gain de temps. Ainsi, le processus de distribution n'est pas retardé par la mise en rotation du plateau 320b pendant l'étape de distribution du produit par la tête.

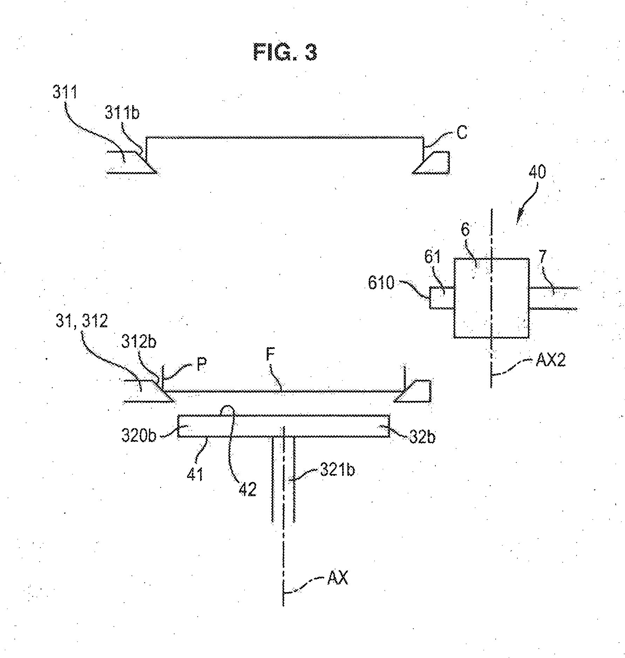

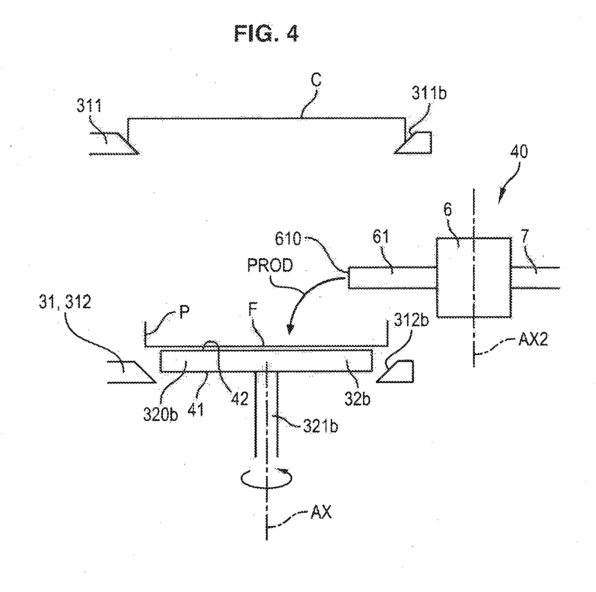

Des moyens de synchronisation sont par exemple prévus pour synchroniser le passage de la tête 6 dans la position de distribution et l'envoi de produit dans l'embout 61 avec la mise en rotation du plateau 320b. Le plateau 320b est mis en rotation au moins pendant la durée de distribution de produit par l'embout 61 de la tête 6 en position de distribution. La tête 6 est mobile entre une position d'escamotage représentée à la figure 3, dans laquelle l'embout n'est pas au-dessus du fond F et du plateau 320b et où le produit n'est pas distribué par la tête 6, et la position de distribution représentée aux figures 1 et 4, par exemple par rotation autour d'un autre axe vertical AX2. Des moyens automatiques de commande sont prévus pour déplacer la tête 6 entre l'une et l'autre de ces positions.

Dans la position de distribution, l'extrémité 610 de l'embout 61 se trouve par exemple au-dessus d'un point du plateau 320b distant de l'axe géométrique AX de rotation autour duquel tourne le plateau 320b, afin, par la force centrifuge due à la rotation, que le produit déversé ainsi que représenté par la flèche PROD s'éloigne du centre situé sur l'axe de rotation. Du fait de la vitesse horizontale de sortie du produit hors de l'extrémité 610, c'est la zone de retombée du produit sur le fond F qui peut être distante de son centre situé sur l'axe AX de rotation ainsi que représenté par la flèche PROD représentant la trajectoire du produi déversé par la tête 6.

La vitesse de rotation du plateau est par exemple comprise entre 10 et 100 tours par minute et notamment entre 50 et 90 tours par minute. Dans un mode de réalisation, la vitesse de rotation du plateau 320b est par exemple de 75 tours par minute pour le produit formé par de la gélose.

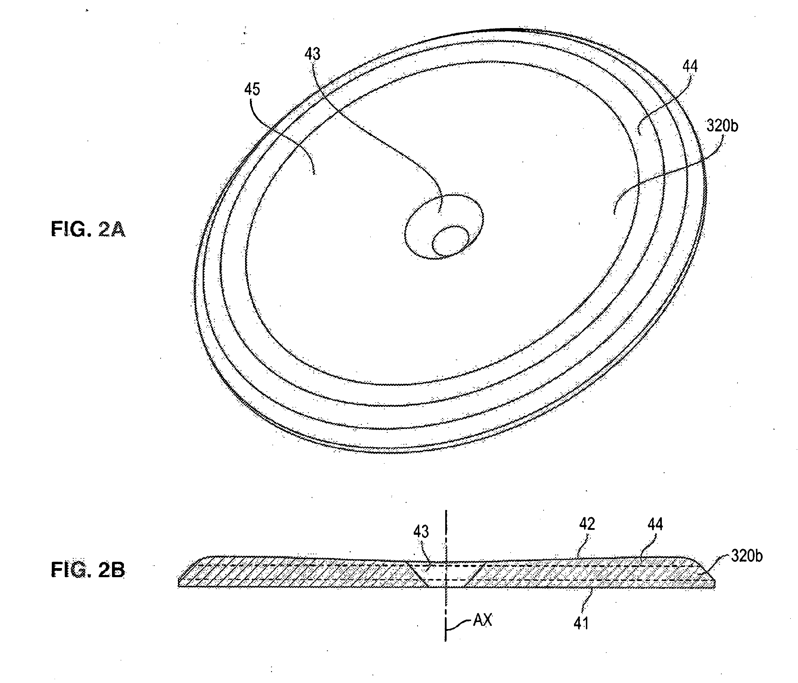

Dans le mode de réalisation représenté aux figures 2A et 2B, la surface supérieure 42 du plateau 320b, servant à recevoir la surface inférieure du fond F, est par exemple légèrement concave de sa périphérie vers son centre.

En effet, on s'est aperçu que les fonds F de boites de Petri pouvaient avoir sur la surface inférieure un léger bombement central vers le bas par lequel elles reposent obligatoirement sur toute surface rigoureusement plane. Dans ce cas, seul un autre point périphérique du fond F repose également, en plus du bombement central sur la surface plane. Ces contacts ponctuels du fond F avec une surface plane jouent en la défaveur d'un entraînement en rotation par un plateau rigoureusement plan. La forme concave de la surface supérieure 42 du plateau 320b permet de loger la partie centrale bombée vers le bas du fond F au centre du plateau 320b et d'assurer que la surface de contact du fond F avec le plateau 320b s'étende sur toute la périphérie inférieure du fond F. A cet effet, la surface 42 concave supérieure du plateau 320b comporte par exemple un évidement 43 pour recevoir le centre d'un fond F de boîte de Petri. L'axe AX de rotation vertical sur lui-même du plateau 320b passe sensiblement par cet évidement 43. L'évidement 43 va par exemple de la surface supérieure 42 à la surface inférieure 41 du plateau 320b et forme donc un trou traversant. La surface 42 présente donc une pente positive du centre du plateau 320b vers sa périphérie 44.

Le plateau 320b est fixé sur la broche 321b par exemple par une vis 47 logée dans le trou 43 par exemple tronconique se rétrécissant de haut en bas pour retenir la vis 47 à l'intérieur du trou 43.

La surface périphérique supérieure 44 du plateau 320b est par exemple en un matériau distinct de sa partie 45 qu'elle entoure, et par exemple en un matériau assurant une meilleure adhérence par contact avec le fond F d'une boîte de Petri que la partie 45. La partie 44 est par exemple en un matériau caoutchouteux, tandis que la partie 45 est par exemple métallique.

Par conséquent, dans le cas général, le fond F d'une boîte de Petri repose en majeure partie par une surface périphérique sur le plateau 320b, afin d'améliorer l'entrainement en rotation.

Le plateau 320b est moins large que le fond F de la boîte P de Petri.

Aux figures 3 et 4 et aux figures suivantes, le dispositif d'amenée du fond F au poste de distribution comporte une navette 31 comporte une platine supérieure 311 de support du couvercle C de la boîte P de Petri et une platine inférieure 312 de support F de la même boîte P de Petri. La platine 311 comporte une deuxième ouverture supérieure 311b qui sert au passage du fond F de la boîte de Petri et à retenir le couvercle C d'une boîte de Petri. La deuxième ouverture supérieure 311b possède une largeur Lb supérieure à celle du fond F d'une boîte de Petri, mais inférieure à celle de son couvercle C. La deuxième ouverture inférieure 312b sert au support du fond F de la boîte de Petri et possède une largeur inférieure à celle de ce fond F. Les deuxièmes ouvertures 311 b et 312b sont donc appelées ouvertures de support d'une boîte P de Petri.

Aux figures 1, 3 et 4, la navette 31a amené le fond F et le couvercle C jusqu'au poste 40 de distribution et se trouve dans une position d'arrêt au poste 40 de distribution, appelée dans ce qui suit deuxième position d'arrêt. Lorsque la navette 1 arrive dans cette deuxième position d'arrêt au poste 40 de distribution, la tête 6 de distribution se trouve initialement dans la position d'escamotage représentée à la figure 3, le fond F repose sur la deuxième ouverture inférieure 312b de la platine 312, le couvercle C repose sur la deuxième ouverture supérieure 311b et le piston 32b est dans une position abaissée pour avoir son plateau 320b sous la platine inférieure 312. Puis, le plateau 320b du piston 32b est soulevé au travers de l'ouverture inférieure 312b par des moyens d'entrainement vertical de sa broche 321b pour soulever le fond F dans une première position d'élévation à distance au-dessus de cette ouverture inférieure 312b ainsi que représenté à la figure 4. Une fois le piston dans cette première position d'élévation, le plateau 320b est mis en rotation sur lui-même ainsi que décrit ci-dessus. En même temps ou après le soulèvement du piston 32b, la tête 6 est déplacée de la position d'escamotage à la position de distribution représentée à la figure 4 pour ensuite déverser du produit dans le fond F mais en rotation par le plateau 320b tournant. Puis, après arrêt du déversement du produit par la tête, la tête 6 passe de la position de distribution à la position d'escamotage se trouvant à l'écart de la course du fond F et du plateau 320b. Le plateau 320b soutenant le fond F contenant le produit P ayant été déversé est alors déplacé vers le haut depuis la position d'élévation de la figure 4 pour traverser la deuxième ouverture supérieure 311b de la platine 311 et rentré de bas en haut dans le couvercle C s'y trouvant, afin de refermer le couvercle C sur le fond F. Le couvercle C ainsi refermé sur le fond F est ensuite encore élevé par le plateau 320b jusqu'à une colonne de réception ainsi que cela sera décrit ci-dessous. Dans la position d'escamotage, l'embout 61 et son extrémité 610 de sortie se trouvent au-dessus d'un bac ou renfoncement 48 collecteur, prévu pour recueillir les gouttes qui tombent éventuellement de l'embout 61 après la distribution dans le fond F, évitant ainsi de salir le reste du dispositif et notamment tous ses mécanismes servant au déplacement de la navette 31, du ou des pistons et de la tête 6.

On décrit maintenant ci-dessous d'autres parties d'un mode de réalisation du dispositif de distribution. Dans ce qui suit, le piston 32b est le deuxième piston 32b, le plateau 320b est le deuxième plateau 320b.

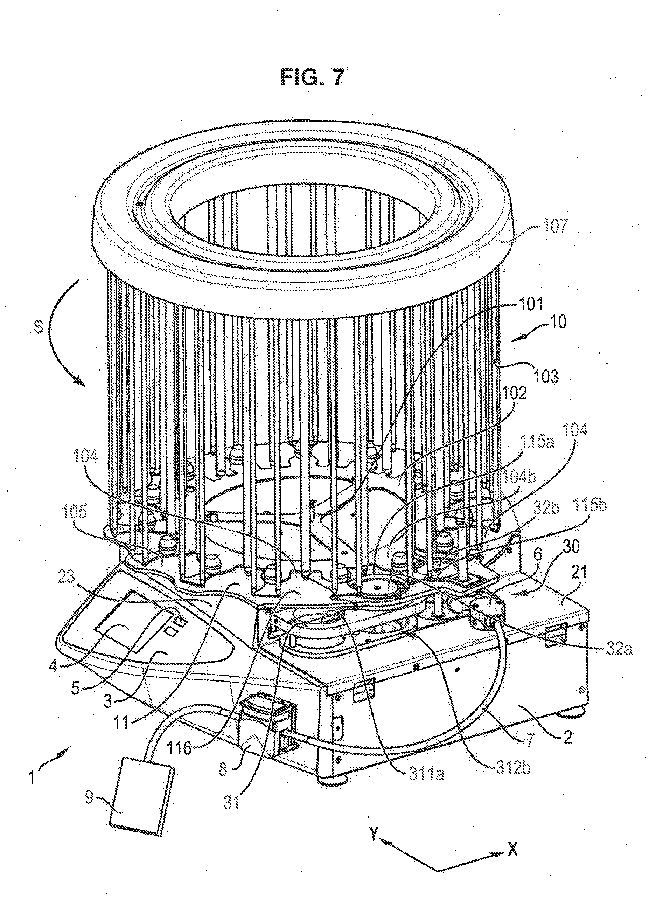

Aux figures 5, 6A, 6B, 7, 8, 9A, 9B, 9C, 9D, 9E, 9F, 9G, 10A, 10B, 10C, 10D, le dispositif 1 de distribution comporte un socle 2 se présentant par exemple sous la forme d'un caisson. Le socle 2 comporte une face avant 22 sur laquelle se trouve un panneau de commande du dispositif, muni par exemple d'un écran de contrôle 4 et d'un ou plusieurs boutons 5 de commande ou toute autre interface 5, permettant à l'utilisateur de commander le fonctionnement automatique du dispositif 1. Sur le socle 2 est prévu le moyen 6 de distribution d'un produit prescrit qui est par exemple de la gélose liquide, ou un milieu gélosé. Ce moyen 6 de distribution est par exemple sous la forme d'une tête 6 rotative autour d'un axe vertical sur une face supérieure 21 du socle 2, la tête 6 comportant la buse ou embout 61 de sortie du produit. Ce moyen 6 de distribution est alimenté en produit par un tuyau 7 relié à un moyen 8 d'envoi du produit vers le moyen 6, ce moyen 8 étant par exemple formé par une pompe péristaltique 8 disposée sur le socle 2, par exemple sur sa face avant 22, l'autre extrémité du tuyau 7 étant reliée à une réserve 9 de produit.

Le socle 2 comporte une partie supérieure 23 surélevée par rapport à la face supérieure latérale 21, adjacente à cette partie supérieure 23. La partie surélevée 23 supporte un carrousel 10 de stockage de boîtes de Petri. Le carrousel 10 est par unique sur le socle 2 et est rotatif par rapport à un axe vertical central 101 sur la partie 23 du socle 2. Le carrousel 10 comporte un anneau inférieur 102 supportant des moyens 103 de stockage de boîtes de Petri en piles. Ces moyens 103 délimitent une pluralité de colonnes verticales 104 de logement et de guidage de piles de boîtes de Petri et comprennent à cet effet des tiges 103 ou tout autre moyen de guidage vertical de boîtes de Petri. Un anneau supérieur 107 amovible est prévu pour coiffer les moyens 103. Chaque colonne 104 débouche vers le bas par une ouverture 105 de passage d'une boîte de Petri dans l'anneau inférieur 102. Par conséquent, ces ouvertures 105 ont un diamètre supérieur à celui des boîtes de Petri. Les ouvertures 105 des colonnes 104 sont réparties à équidistance de l'axe central 101 de rotation et de manière équiangle sur l'anneau inférieur 102. Les ouvertures 105 par exemple périphériques à l'anneau inférieur 102 et sous la forme d'échancrures de l'anneau 102. La hauteur des moyens 103 de guidage est prévu pour la réception dans chaque colonne 104 d'une pile de plusieurs boîtes de Petri en un nombre prescrit jusqu'à l'anneau 107. Chaque boîte P de Petri est introduite dans une colonne 104 en ayant son couvercle C posé sur son fond F pour que la boîte soit fermée de manière amovible, les boîtes P de Petri ayant de manière connu un couvercle C plus large que leur fond F, ainsi que cela est représenté aux figures. Lorsque la boîte de Petri est fermée, le rebord du couvercle se trouve à l'extérieur du rebord du fond.

Sous l'anneau inférieur 102 du carrousel et sur le socle 2 se trouve une plaque 11 de guidage horizontal des boîtes de Petri lorsqu'elles sont dans le carrousel 10. L'axe 101 du carrousel est relié à des moyens d'entrainement en rotation situés dans le socle 2 pour faire tourner horizontalement le carrousel 10 autour de cet axe vertical 101. La plaque 11 de guidage horizontal est fixée sur la partie surélevée 23 et comporte une partie 116 située au-dessus de la surface supérieure 21 et sous plusieurs colonnes 104 du carrousel 10. La plaque 11 de guidage horizontal comporte dans la partie 116 une première ouverture ou passage 115a et une deuxième ouverture ou passage 115b ayant la même disposition spatiale que deux ouvertures inférieures 105 du carrousel 10, pour permettre le passage d'une boîte de Petri au travers de ces ouvertures 105, 115a, 115b. Pour plus de compacité, l'écartement entre la première ouverture 115a et la deuxième ouverture 115b de la plaque 105 correspond à l'écartement entre deux première et deuxième ouvertures inférieures respectives adjacentes 105a et 105b du carrousel 10.

Les première et deuxième ouvertures 115a et 115b de la plaque 11 sont situées au-dessus d'une station 20 permettant à la fois le dépilement des boîtes de Petri vides, leur ouverture, la distribution du produit dans leur fond, leur fermeture et leur ré-empilement à l'état rempli. La station 20 se situe sur la face supérieure 21 du socle 2.

La station 20 comporte un système 30 de déplacement des boîtes de Petri une à une à l'état vide depuis le dessous de la colonne 104b dans l'ouverture 105b par l'ouverture 115a, par l'intermédiaire d'un organe 31 de transfert en translation horizontale et de deux premier et deuxième pistons 32a, 32b à déplacement vertical sous respectivement les deuxième et première colonnes 104a, 104b. Chaque piston 32a, 32b comporte un plateau supérieur 320a, 320b, fixé à une tige verticale inférieure 321a, 321b reliée dans le socle 2 à des moyens d'entrainement en déplacement vertical pour faire monter et descendre les pistons 22a, 22b.

L'organe 31 de transfert est une navette 31 effectuant un aller-retour en translation suivant une direction horizontale longitudinale X.

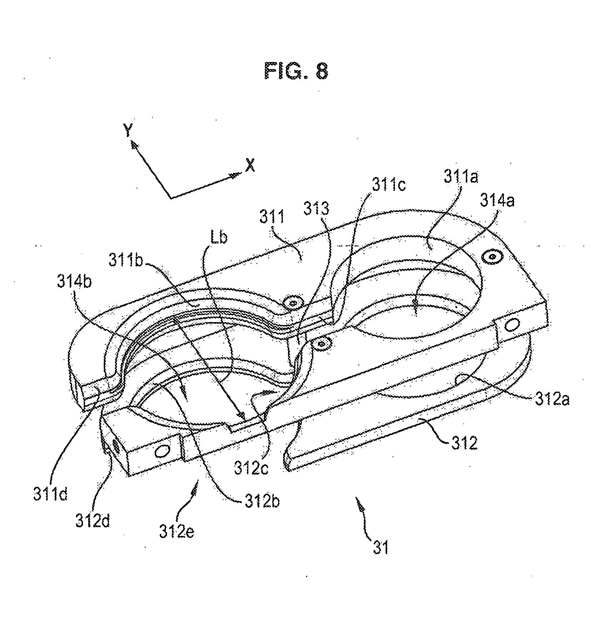

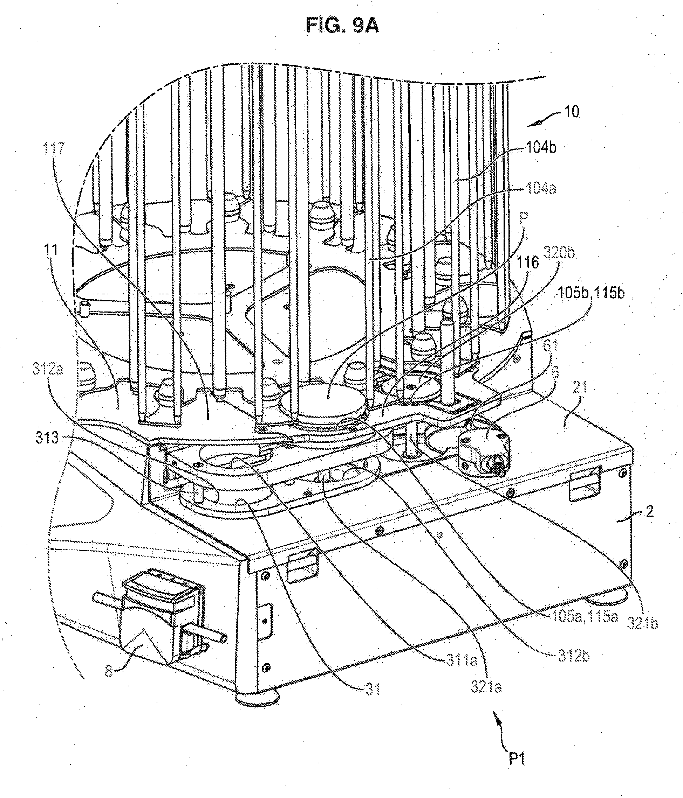

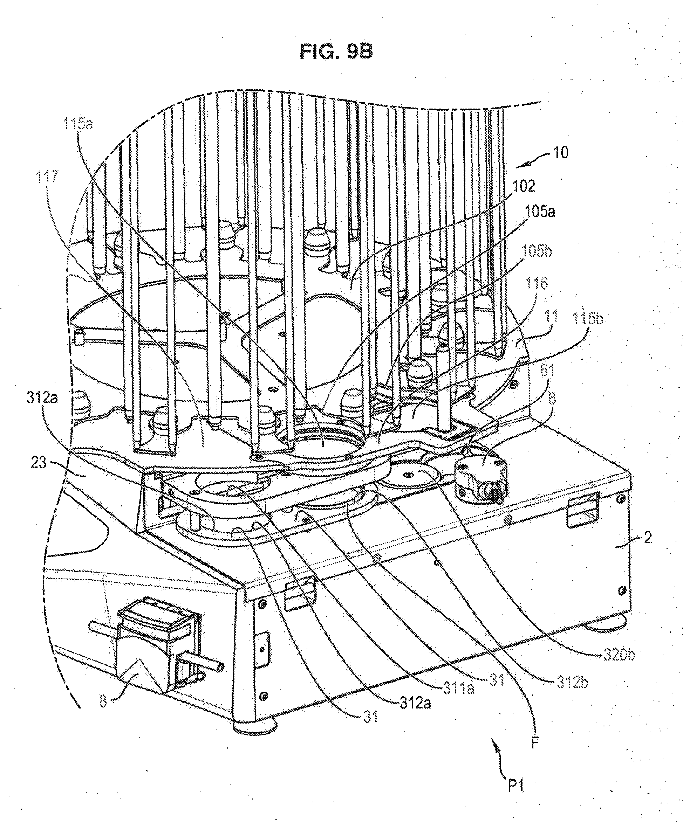

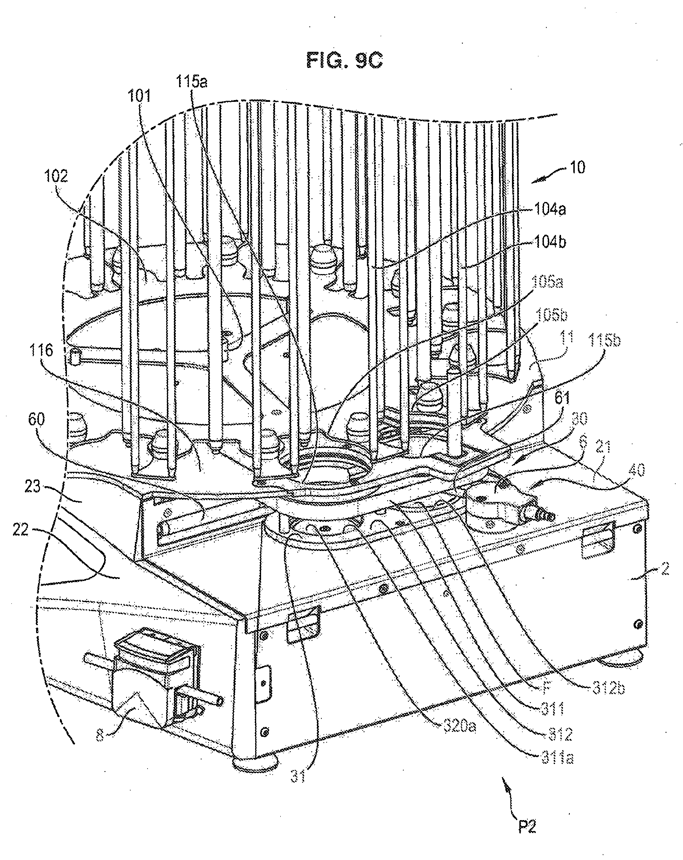

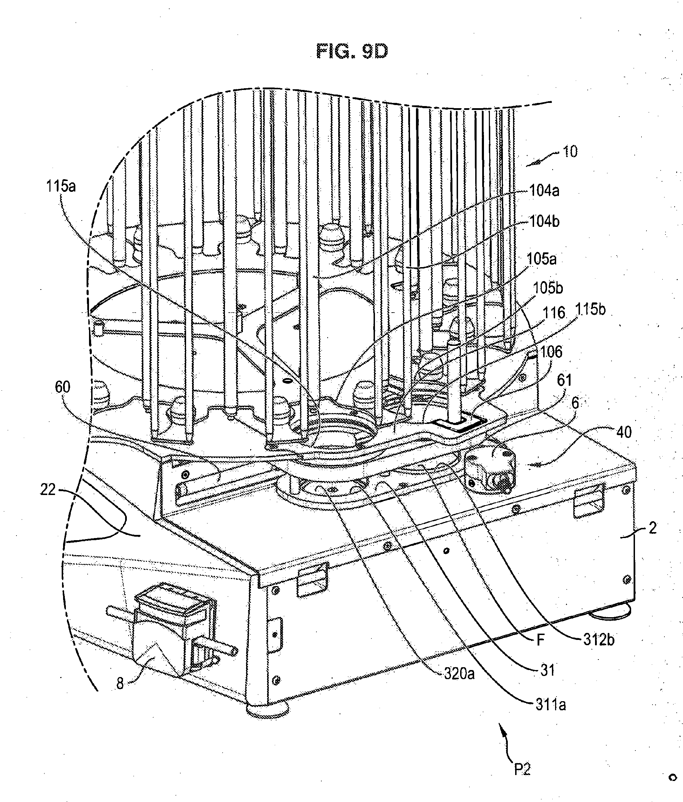

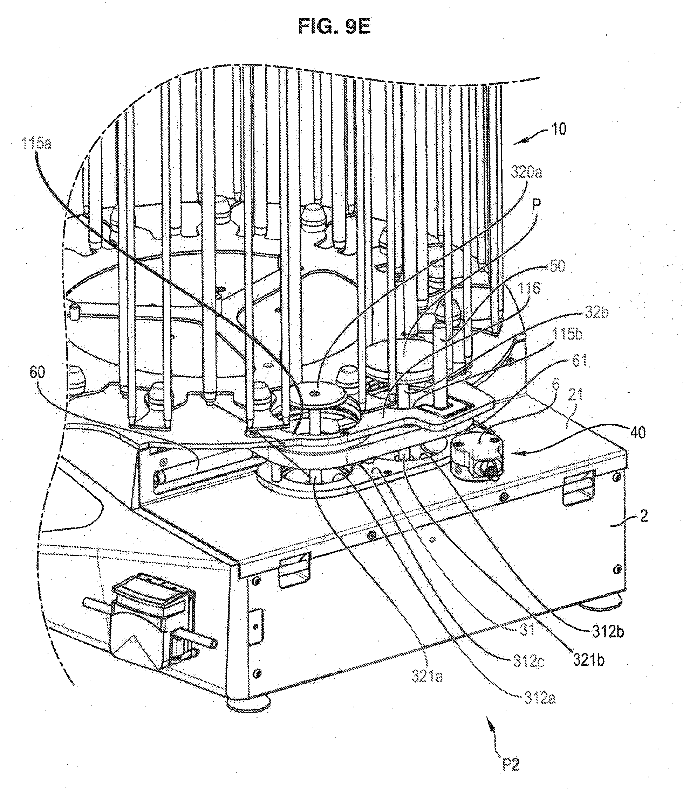

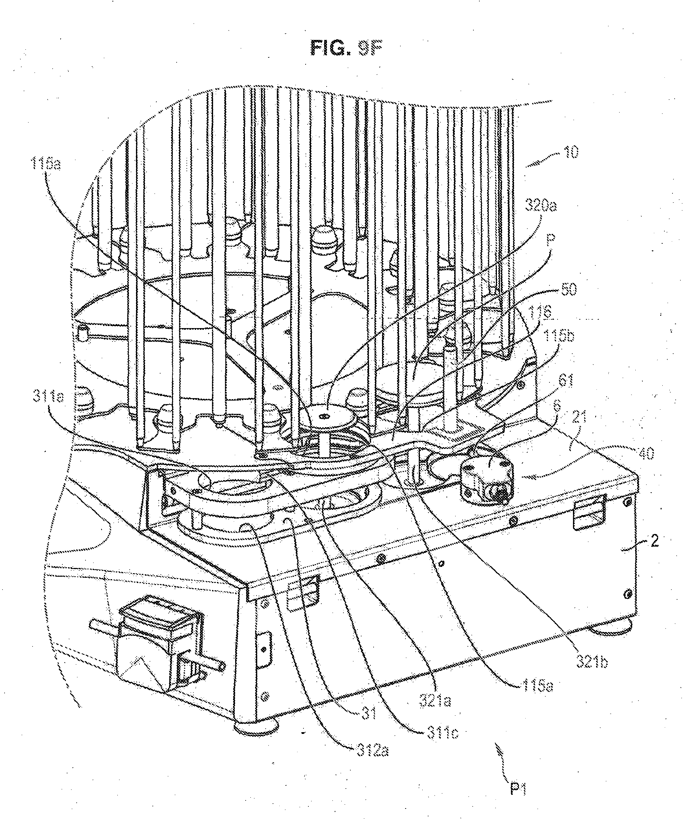

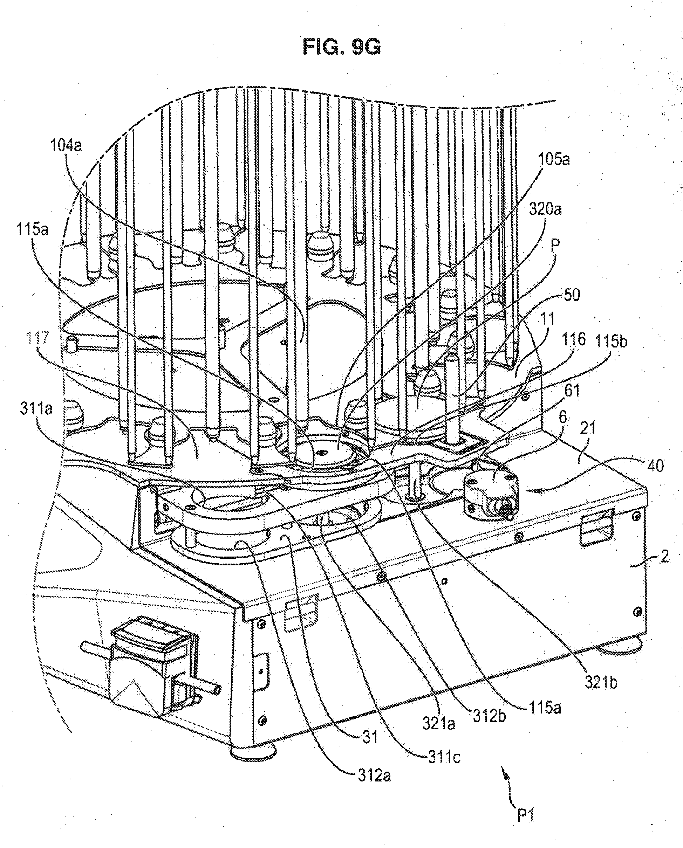

Un mode de réalisation de l'organe 31 est représenté à la figure 8 et est décrit plus en détail ci-dessous. Dans ce mode de réalisation, ainsi que représenté aux figures 9A, 9B, 9C, 9D, 9E, 9F et 9G, les pistons 32a et 32b ont des mouvements de translation verticale simultanée, de manière à ce que leurs plateaux 320a, 320b se trouvent à la même hauteur l'un de l'autre. Ceci permet dans ce cas de n'avoir qu'un seul moteur d'entraînement en translation verticale pour les deux pistons 32a et 32b. Dans le mode de réalisation représenté, les premier et deuxième pistons 32a et 32b présentent le même mouvement en synchronisme, et se trouvent en même temps en position haute (figures 9A et 9G), en positon basse (figures 9B, 9C, 9D) ou en position d'élévation (figures 9E, 9F).

La navette 31 comporte une platine supérieure 311 et une platine inférieure 312 solidaires l'une de l'autre au moyen d'entretoises 313. Bien entendu, la navette 31 peut être réalisée en une seule pièce. La platine inférieure 312 sert au guidage en translation de la navette 31 sur la surface 21 et à la stabilité de la navette 21 sur cette surface 21. Les moyens 60 d'entraînement en translation de la navette 31 sont latéraux et logés dans le socle 2 sous la partie surélevée 23.

Aux figures, la platine supérieure 311 comporte une première ouverture supérieure 311a et la deuxième ouverture supérieure 311b, qui sont situées respectivement au-dessus d'une première ouverture inférieure 312a et de la deuxième ouverture inférieure 312b de la platine inférieure 312. Les premières ouvertures inférieure et supérieure 311a et 312a servent au passage du premier plateau 320a du premier piston 32a et sont plus larges que ce dernier.

En outre, la platine supérieure 311 comporte un passage 311 c de liaison entre la première ouverture supérieure 311a et la deuxième ouverture supérieure 311b, pour permettre le passage de la tige 321a du premier piston 32a entre ces ouvertures 311a et 311b. Le passage 311c possède une largeur horizontale transversale supérieure à celle de la tige 321a du premier piston 32a. La platine inférieure 312 comporte un passage 312c de liaison entre la première ouverture inférieure 312a et la deuxième ouverture inférieure 312b, pour permettre le passage de la tige 321a du premier piston 32a entre ces ouvertures 31 la et 311b. Le passage 312c possède une largeur horizontale transversale supérieure à celle de la tige 321a du premier piston 32a.

A la figure 8, la navette 31 s'étend suivant la direction horizontale longitudinale X qui est la direction horizontale allant entre les deux ouvertures 115a et 115b de la plaque 105. La direction horizontale transversale Y est celle perpendiculaire à la direction longitudinale X.

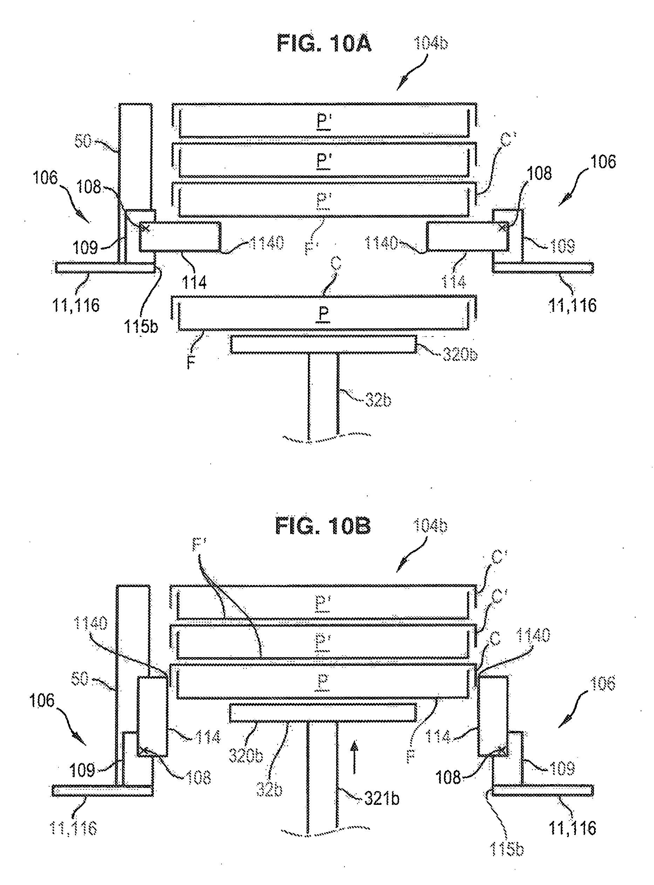

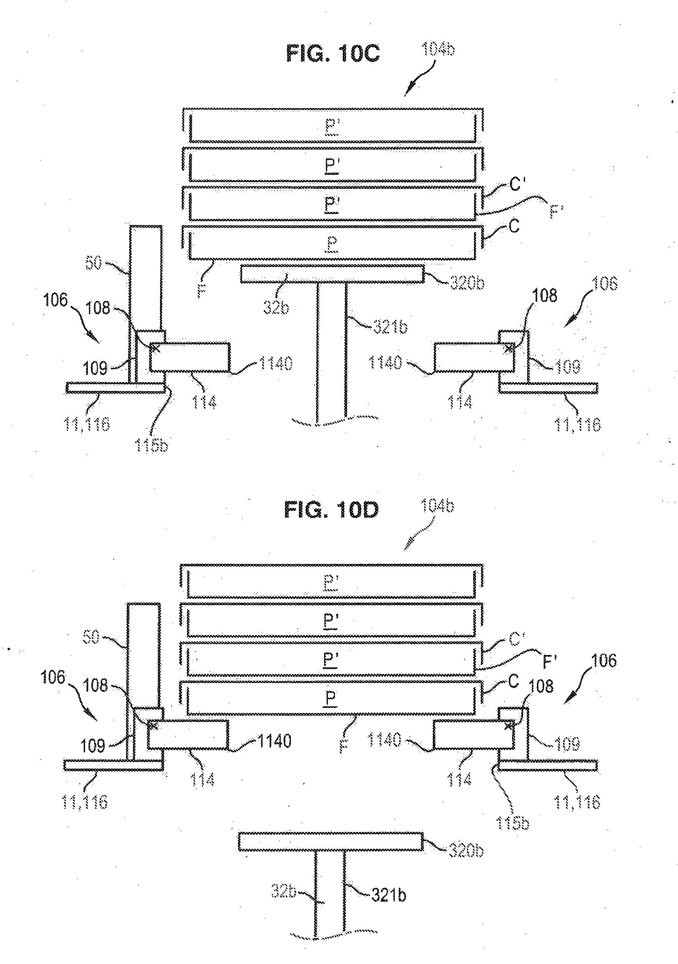

La plaque 11 comporte des moyens 106 de retenue et de passage des boîtes P, P' de Petri empilées au-dessus de la deuxième ouverture 115b. Ces moyens 106 sont mobiles entre une première position de retenue d'une pile de boîte P' de Petri par le dessous au-dessus de l'ouverture 115b dans la colonne 104b, ainsi que cela est représenté aux figures 10A, 10C et 10D, et une deuxième position de libération de passage des boîtes de Petri dans l'ouverture 115b, ainsi que cela est représenté à la figure 10B. Ces boîtes P' se trouvant déjà dans la colonne 104b comportent un couvercle C' posé sur un fond F'. Les moyens 106 comprennent par exemple en plusieurs endroits autour de l'ouverture 115b une patte 114 articulée par un axe 108 de rotation horizontale sur un support 109 fixé à la partie 116 à proximité de l'ouverture 115b, par exemple deux pattes 114 diamétralement opposées. Dans la position de retenue des figures 9B, 9C, 9D et 10A, les pattes 114 s'étendent au-dessus de l'ouverture 115b pour délimiter entre eux une largeur inférieure à celle du fond F' des boîtes de Petri P' pour soutenir le fond F' de celle du bas, cette largeur permettant le passage du plateau 320b du piston 32b entre les pattes 114 et étant supérieure à la largeur de ce plateau 320b.

Lorsque le plateau 320b ayant dessus une boîte P de Petri ayant son couvercle C posé sur son fond F traverse de bas en haut l'ouverture 115b vers la colonne 104b à la figure 10B, le couvercle C de cette boîte B, soulevé par le plateau 320b, soulève les pattes 114 pour les faire tourner vers l'extérieur. Au cours de la montée du plateau 320b à la figure 10B, le plateau 320b est à une hauteur telle par rapport à l'ouverture 115b que les pattes 114 sont en appui par leur extrémité libre 1140 éloignée de l'axe 108 latéralement contre les boîtes P, P' de Petri. Des moyens sont prévus pour contraindre les pattes 114 à être appliquées vers la position de retenue, par exemple par gravité ou du fait du positionnement de leur axe 108 dans la patte 114, ou par un ressort ou autres moyen positif de rappel.

A la figure 10C, le plateau 320b passe dans une deuxième position d'élévation au-dessus de l'extrémité libre 1140 qui était appuyée contre les boîtes P, P' soutenues par le plateau. Cette deuxième position d'élévation correspond aux figures 9E et 9F. Par conséquent, l'extrémité 1140 des pattes retombe dans la position de retenue vers l'ouverture 115b. La deuxième position d'élévation de la figure 10C est au-dessus de la première position d'élévation représentée à la figure 4.

Lorsque de la figure 10C à la figure 10D, le piston 32b redescend, son plateau 320b passe entre les pattes 114 se trouvant en position de retenue, puis traverse l'ouverture 115b, ce qui dépose la pile de boîtes P, P' sur les pattes 114 en position de retenue.

Le fonctionnement du dispositif est le suivant.

On a représenté aux figures 9A, 9B, 9C, 9D, 9E, 9F et 9G le trajet d'une boîte P de Petri vide, c'est-à-dire comportant un couvercle C et un fond F, qui est celle située la plus en bas de la colonne 104a.

Aux figures 5, 9A, 9B, 9F et 9G, la navette 31 se trouve dans une première position d'arrêt.

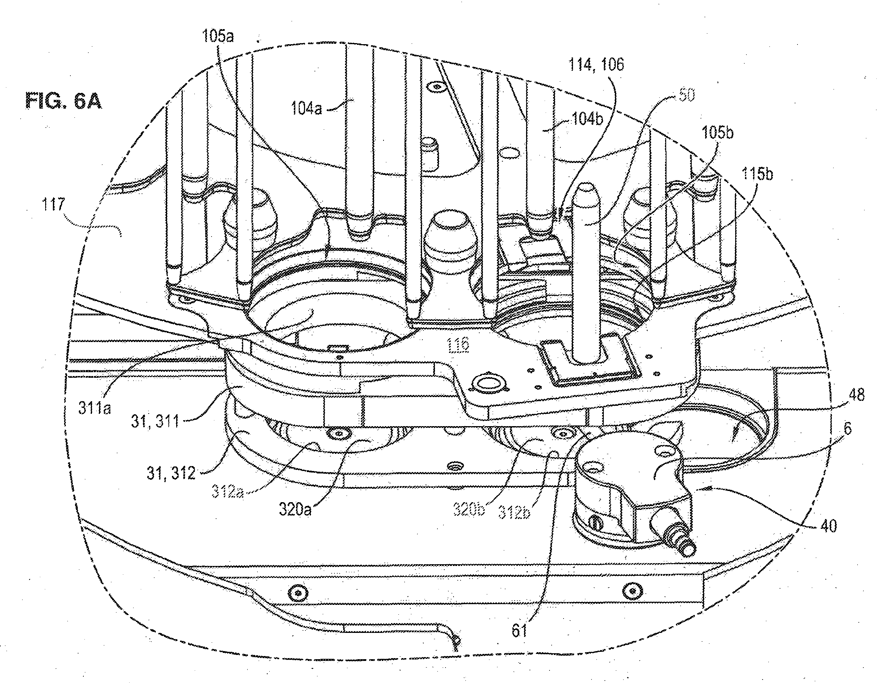

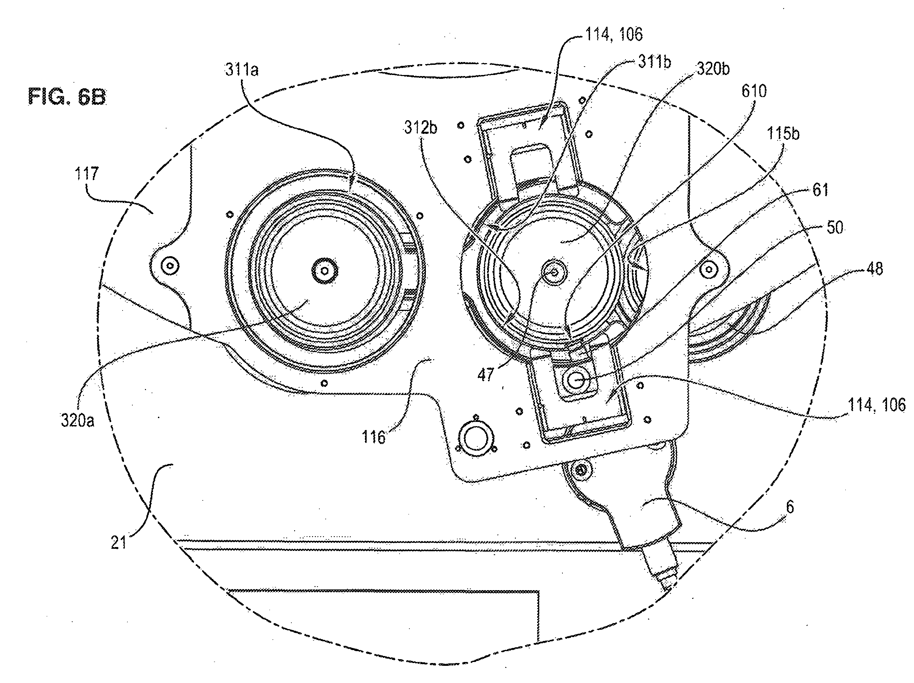

Aux figures 6A, 6B, 9C, 9D et 9E, la navette 31 se trouve dans une deuxième position d'arrêt.

Dans la première position P1 d'arrêt, les ouvertures de support 311b et 312b de la navette 31 se trouvent sous la première ouverture 115a, se trouvant elle-même sous une ouverture 105a d'une colonne 104a de boîtes de Petri vides et fermées. Dans la première position P1 d'arrêt, la navette 31 est disposée de l'autre côté du deuxième piston 32b, une partie pleine 117 de la plaque 11 étant interposée entre le carrousel 10 et la première ouverture 311a supérieure de la navette 31.

Dans la deuxième position P2 d'arrêt, les premières ouvertures 311a et 312a de la navette 31 se trouvent sous la première ouverture 115a et sous la première colonne 104a. Dans la deuxième position d'arrêt, les deuxièmes ouvertures 311 b et 312b de la navette 31 se trouvent sous la deuxième ouverture 115b et sous la deuxième colonne 104b.

Dans le mode de réalisation représenté aux figures, la navette 31 comporte comme position d'arrêt, uniquement la première position P1 d'arrêt et la deuxième position P2 d'arrêt.

A la figure 9A, le premier plateau 320a du premier piston 32a soutient la première pile 104a de boîtes P de Petri dans la première colonne 104a au-dessus des deuxièmes ouvertures 312b et 311b, la tige 321a passant dans ces ouvertures 312b et 311 b.

Puis, le piston 32a est abaissé au-travers des deuxièmes ouvertures 311b et 312b de la navette 31 pour amener le couvercle C de la boîte P de Petri du bas de la colonne 104a sur la deuxième ouverture supérieure 311b et pour amener le fond F de la boîte P sur la deuxième ouverture inférieure 312b, pour arriver dans la position représentée à la figure 9B. Aux figures 9B, 9C, 9D, les plateaux 320a, 320b des pistons se trouvent sous la navette 31, par exemple dans des logements ménagés dans la surface 21.

Aux figures 9A et 9B, le moyen 6 de distribution de produit se trouve dans la position d'escamotage où la buse 61 ne se trouve pas au-dessus de l'ouverture 312b ni au-dessus du piston 32b. Dans cette position d'escamotage, l'embout 61 laisse libre le passage entre la deuxième ouverture inférieure 312b et la deuxième ouverture supérieure 311b et se trouve dans ce cas en dehors de la course du piston 32b.

Puis, la navette 31 est déplacée par translation horizontale longitudinale suivant la direction X pour arriver dans la deuxième position d'arrêt représentée à la figure 9C. Le moyen 6 de distribution passe à la position de distribution pour le déversement du produit, par exemple par rotation de la tête 36 de distribution de manière à amener son embout 61 de déversement entre les platines 311 et 312 au-dessus de la deuxième ouverture inférieure 312b et donc au-dessus du fond F s'y trouvant à la figure 9C.

Le fond F supporté sur/dans la deuxième ouverture inférieure 312b et le couvercle C supporté sur/dans la deuxième ouverture supérieure 311b se trouvent sous la deuxième ouverture 115b de la plaque fixe 11 et sous la deuxième colonne 104b. Des moyens automatiques prévus dans le socle 2 provoquent alors la commande du moyen 8 d'envoi pour faire parvenir du produit dans le moyen 6 de distribution, ce produit étant alors déversé par ce moyen 6 au-dessus de la deuxième ouverture supérieure 312b dans le fond F de boîte de Petri s'y trouvant.

Puis, dans la position représentée à la figure 9D, le moyen 6 de distribution est remis dans sa position écartée de la deuxième ouverture inférieure 312b ou position d'escamotage.

Puis, à la figure 9E, les pistons 32a et 32b passent dans la position d'élévation de la figure 10C au-dessus de la navette 31 et dans la colonne 104b. Le deuxième piston 32b soulève donc d'abord le fond F se trouvant dans la deuxième ouverture inférieure 312b, puis remonte le fond F dans le couvercle C se trouvant dans la deuxième ouverture supérieure 311b, ce qui ferme la boîte P de Petri dont le fond contient du produit ayant été déverse dedans par le moyen 6, puis soulève la boîte P ainsi fermée et remplie dans la deuxième colonne 104b au travers de la deuxième ouverture 115b et de la deuxième ouverture 104b du carrousel 10. Le plateau 320b soutient donc la boîte P fermée et remplie dans la colonne 104b et les autres boîtes P' qui se trouvaient éventuellement déjà dans la colonne 104b.

Ensuite, à la figure 9F, la navette 31 est translatée dans sa première position d'arrêt correspondant à la figure 9B. A la figure 9F, les deuxièmes ouvertures 312b et 311b de la navette 31 se trouvent sous les premières ouvertures 115a et 104a, tandis que les premières ouvertures 311a et 312a se trouvent sous la partie pleine 117 de la plaque 11. Au cours de ce mouvement de translation, la tige 321a du premier piston 32a passe donc des deuxièmes ouvertures 311b et 312b aux premières ouvertures 311a et 312a en traversant les passages 311c et 312c ménagés dans les platines 311 et 312.

Puis, à la figure 9G, les plateaux 320a et 320b des pistons reviennent à une position plus basse que la position d'élévation de la figure 9F, cette position de la figure 9G correspondant à celle des figure 9A et 10D. Aux figures 9A et 9G, les boîtes de Petri de la colonne 104b sont soutenues par les moyens 106.

Un moyen 50 de guidage vertical des boîtes P, P' dans la deuxième colonne 104b est fixé sur la plaque 11 à proximité de l'ouverture 115b, et comprend par exemple une barre verticale 50 pour mettre les boîtes P, P' suivant un empilement droit lors de la montée et de la descente du deuxième piston 32b aux figures 10A à 10D.

L'encombrement de la navette 31 est d'environ deux boîtes de Petri juxtaposées correspondant à son positionnement sous deux colonnes 104 adjacentes comme à la figure 9C. La course de la navette 31 correspond dans le mode de réalisation représenté à la distance entre les deux colonnes 104a et 104b de boîtes de Petri adjacentes.

La largeur de la première ouverture supérieure 311a est plus petite que la largeur du fond F d'une boîte de Petri P pour soutenir la pile de boîtes de Petri issues de la première colonne 104a lorsque cette première ouverture supérieure 311a se trouve dessous aux figures 9C et 9D.

Bien entendu, la navette 31 pourrait avoir toute autre forme que celle représentée aux figures, tout en ayant un premier passage vertical 314a allant de la première ouverture inférieure 312a à la première ouverture supérieure 311 a pour permettre le déplacement du premier plateau 320a du premier piston 32a dans celui-ci, un deuxième passage vertical 314b allant de la deuxième ouverture inférieure 312b à la deuxième ouverture supérieure 311b pour le passage des premier et deuxième plateaux 320a et 320b des premier et deuxième piston 32a et 32b et le troisième passage 311c supérieur entre les ouvertures supérieures 311b et 311a et le quatrième passage inférieur 312c entre les ouvertures inférieures 312a et 312b, ces troisième et quatrième passages 311c et 311d pouvant bien entendu n'être qu'un.

Du fait du faible encombrement de la navette 31, il est possible de réaliser un carrousel 10 ayant un plus grand nombre de colonnes 104. Par conséquent, à nombre de boîtes P de Petri égal, on peut arriver à un carrousel 10 moins haut, du fait de boîtes de Petri réparties sur un plus grand nombre de colonnes 104. La fabrication du dispositif est également plus simple et moins coûteuse.

Pour une utilisation optimale du dispositif, l'utilisateur remplit toutes les colonnes 104 du carrousel sauf une pile de boîtes de Petri vides. La colonne vide de boites de Petri est amenée par rotation du carrousel 10 au-dessus de la deuxième ouverture 115b et au-dessus du plateau 320b du deuxième piston 32b à la figure 9A. Puis on remplit successivement les boîtes P de Petri vides issues de la colonne 104a selon le procédé décrit ci-dessus.

Les boîtes P remplies sont donc transférées l'une après l'autre dans la colonne 104b ne comportant initialement aucune boite. Lorsque la colonne 104a située au-dessus de la première ouverture 115a ne comporte plus de boîtes P, on fait tourner le carrousel 10 dans le sens S représenté à la figue 1 pour faire passer cette nouvelle colonne vide au-dessus de la deuxième ouverture 115b. Ce sens S d'avancée du carrousel d'une colonne va dans le même sens que de la première position P1 d'arrêt à la deuxième position P2 d'arrêt. Dans ce cas, une nouvelle colonne de boîtes de Petri vides, qui était initialement sur la partie pleine 112 de la plaque 11, se retrouve au-dessus de la première ouverture 115a de dépilement. On recommence alors le même processus avec cette pile de boîtes.

La navette 31 comporte également un passage d'extrémité longitudinal pour la tige 321 b du piston, c'est-à-dire dans le mode de réalisation représentée, un premier passage d'extrémité longitudinal supérieur 311d dans la platine supérieure 311, débouchant d'une part sur la deuxième ouverture supérieure 311b et d'autre part sur l'extérieur dans le sens longitudinal et d'autre part un passage d'extrémité longitudinal inférieur 312d dans la platine inférieure 312, faisant communiquer la deuxième ouverture inférieure 312b avec l'extérieur dans le sens longitudinal. Par exemple, à la figure 8, la deuxième ouverture inférieure 312b et le passage 312d sont sous la forme d'une échancrure de la platine inférieure 312 débouchant vers l'extrémité longitudinale inférieure et vers un côté 312e.

Un détecteur de présence d'un fond F dans la deuxième ouverture inférieure 312b peut être prévu. Ce détecteur est par exemple fixe par rapport au socle 2 pour détecter la présence du fond F dans la deuxième ouverture inférieure 312b dans la première position d'arrêt et/ou dans la deuxième position d'arrêt. Il peut donc être prévu un premier détecteur sous la première colonne 104a. Il peut être également prévu un autre détecteur sous la deuxième colonne 104b. Le processus de remplissage des boîtes P est effectué tant que le détecteur fournit une information de présence de fond F de boîte dans l'ouverture 312b. Dans le cas où le détecteur ne fournit pas une information de présence de fond F de boîte dans l'ouverture 312b, le processus est interrompu ou l'envoi de produit dans le moyen 6 de distribution est interrompu.

Les moyens de commande et de coordination, pour commander les différents moyens d'entraînement du carrousel 10, des pistons 32a, 32b, de la tête 6 et de la navette 31, sont automatisés et actionnés par l'interface 5 de commande. Ils sont prévus par exemple sous la forme d'un microprocesseur dans le socle 2.

Le dispositif suivant l'invention est destiné à être intégré à un automate de remplissage de boîtes de Petri.

Suivant un mode de réalisation, le dispositif comporte
au moins des première et deuxième colonnes (104a, 104b) de guidage vertical de boîtes (P) de Petri, aptes à recevoir respectivement des première et deuxième piles de boîtes de Petri à couvercle disposé sur le fond et aptes à être positionnées respectivement au-dessus d'un premier passage inférieur (115a) de boîte de Petri et au-dessus d'un deuxième passage inférieur (115b) de boîtes de Petri, les premier et deuxième passages (115a, 115b) étant prévus sur une partie fixe (3) par rapport au socle (2),
un système (30) de déplacement d'au moins une boîte de Petri de la première colonne (104a) à la deuxième colonne (104b), comportant :
- un organe (31) de transfert ayant une ouverture supérieure (311b) de support du couvercle (C) d'une boîte de Petri au-dessus d'une deuxième ouverture inférieure (312b) de support du fond (F) d'une boîte de Petri,
- un moyen (6) de distribution pour distribuer ledit produit au-dèssus de l'ouverture inférieure (312b) dans le fond (F) de la boîte de Petri sous l'ouverture supérieure (311b),
- l'organe (31) mobile de transfert étant apte à occuper une première position d'arrêt, où les ouvertures (311b, 312b) de support se trouvent sous le premier passage (115a), et une deuxième position d'arrêt, où les ouvertures (311b, 312b) de support se trouvent sous le deuxième passage (115b),
- un premier piston (32a) comportant un premier plateau (320a) situé sous le premier passage (115a) et apte à traverser les ouvertures (311b, 312b) pour faire descendre le couvercle et le fond d'une boîte de Petri de la première colonne (104a) sur leur ouverture respective (311b, 312b) de support lorsque l'organe (31) de transfert se trouve dans la première position d'arrêt associée,
- un deuxième piston (32b) comportant un deuxième plateau (320b) situé sous le deuxième passage inférieur (115b) et apte à traverser les ouvertures (311b, 312b) pour faire remonter le couvercle et le fond de la boîte de Petri de leur ouverture respective (311b, 312b) de support dans la deuxième colonne (104b), lorsque l'organe (31) de transfert se trouve dans la deuxième position d'arrêt associée,
   l'organe (31) de transfert étant une navette (31) à déplacement en translation par rapport au socle (2) selon un mouvement direct aller et retour des ouvertures (311b, 312b) de support entre l'une et l'autre des première et deuxième positions (P1, P2) d'arrêt,
   le moyen (6) de distribution étant agencé pour distribuer ledit produit au-dessus de l'ouverture inférieure (312b) et sous l'ouverture supérieure (311b) dans le fond (F) de la boîte (P) lorsque ces ouvertures (311 b, 312b) se trouvent dans l'une sélectionnée des première et deuxième positions (P1, P2) d'arrêt.

Suivant un mode de réalisation, le dispositif comporte des moyens d'entraînement synchrone des pistons (32a, 32b) à la même hauteur.

Suivant un mode de réalisation, les pistons (32a, 32b) sont entraînés par le même moteur.

Suivant un mode de réalisation, le dispositif comporte des moyens de coordination des déplacements en translation de la navette (31) et des pistons (32a, 32b) pour les mettre successivement dans les positions suivantes :
- la première position (P1) d'arrêt avec une position d'abaissement du premier plateau (320a) sous l'ouverture inférieure (312b), pour amener le couvercle de la boîte sur l'ouverture supérieure (311b) et amener le fond de la boîte sur l'ouverture inférieure (312b),
- la deuxième position (P2) d'arrêt, avec le premier plateau (320a) sous l'ouverture inférieure (312b) pour la distribution du produit dans le fond (F) de la boîte ouverte située dans l'ouverture inférieure (312b) par le moyen (6) de distribution,
- dans la deuxième position (P2) d'arrêt, une position d'élévation du deuxième plateau (320b) dans la deuxième colonne (104b) pour la fermeture du couvercle sur le fond de la boîte et le transfert de cette boîte fermée dans la deuxième colonne,
- la première position (P1) d'arrêt avec une position d'abaissement du premier plateau (320a) sous l'ouverture inférieure (312b).

Suivant un mode de réalisation, le moyen (6) de distribution comporte une buse (61) de sortie dudit produit, des moyens de déplacement de la buse (6) de distribution étant prévus pour la faire passer entre l'une et l'autre d'une position de distribution dudit produit, où la buse (6) se trouve au-dessus de l'ouverture inférieure (312b) et sous l'ouverture supérieure (311b) dans ladite position sélectionnée d'arrêt, et d'une position d'escamotage où la buse (6) ne se trouve plus entre les ouvertures (311b, 312b), des moyens de commande étant prévus pour n'autoriser le mouvement du piston (32a, 32b) associé à la position sélectionnée d'arrêt que lorsque la buse (6) est position d'escamotage.

Suivant un mode de réalisation, la navette (31) comporte en plus desdites ouvertures (311b, 312b) supérieure et inférieure de support, appelées deuxièmes ouvertures (311b, 312b), un moyen (311b) de soutien de fond (F) de boîtes de Petri muni d'une première ouverture (311b) permettant le traversée du premier piston (32a) dans la première position d'arrêt.

Suivant un mode de réalisation, la navette (31) comporte en plus desdites ouvertures (311b, 312b) supérieure et inférieure de support, appelées deuxièmes ouvertures (311b, 312b), des première ouvertures (311a, 312a) supérieure et inférieure, la navette (31) comporte une platine inférieure (312) et une platine supérieure (311) espacées l'une de l'autre d'une distance prescrite pour ménager un passage du moyen (6) de distribution en position de distribution, la platine inférieure (312) comportant la première ouverture inférieure (312a) de traversée du premier plateau (320a) et du premier piston (32a) et ladite deuxième ouverture inférieure (312b) de support du fond de la boîte, la platine supérieure (311) comportant une première ouverture supérieure (311a) de traversé du premier plateau (320a) et du premier piston (32a) et ladite deuxième ouverture supérieure (311 b) de support du couvercle, la première ouverture supérieure (311a) étant au-dessus de la première ouverture inférieure (312a).

Suivant un mode de réalisation, le dispositif comporte des moyens de montée du premier plateau (320a) du premier piston (32a) au-dessus de la navette (31), le premier plateau (320a) est fixé à au moins une tige inférieure (321a) de liaison du premier plateau (320a) auxdits moyens de montée, la navette (31) comporte au moins un passage (311c, 312c) de communication entre les deuxièmes ouvertures (311b, 312b)et la première ouverture (311a) ou les premières ouvertures (311a, 312a) pour permettre de laisser passer ladite tige inférieure (321a) de soutien lors du déplacement de la navette (31) entre les positions (P1, P2) d'arrêt lorsque le premier plateau (320a) se trouve en position de montée au-dessus de la navette (31).

Suivant un mode de réalisation, le dispositif comporte au moins un carrousel (10) monté rotatif sur un axe vertical du socle (2), le carrousel comportant une pluralité de colonnes (104a, 104b) de guidage vertical de boîtes (P) de Petri, aptes à recevoir chacune une pile de boîtes de Petri à couvercle disposé sur le fond, des moyens d'entraînement en rotation du carrousel (10) étant prévus pour amener deux des colonnes (104a, 104b) respectivement au-dessus du premier passage inférieur (115a) de boîte de Petri et au-dessus du deuxième passage inférieur (115b) pour former lesdites première et deuxième colonnes (104a, 104b).

Suivant un mode de réalisation, le dispositif comporte des moyens (106) de retenue et de passage de boîtes (P, P') de Petri au-dessus du deuxième passage (115b), ces moyens (106) de retenue et de passage étant mobiles entre une première position de retenue des boîtes (P, P') de Petri au-dessus du deuxième passage (115b) dans la deuxième colonne (104b) et une deuxième position de libération de passage des boîtes de Petri dans le deuxième passage (115b), des moyens de contrainte dans le sens allant de la première position de retenue étant prévus,
des moyens de montée du deuxième plateau (320b) du deuxième piston (32b) jusqu'à une position prescrite d'élévation dans la deuxième colonne (104b) étant prévus,
les moyens (106) de retenue étant agencés pour être actionnés dans la deuxième position de libération de passage par butée contre ledit couvercle de la boîte de Petri lorsque le deuxième plateau (320b) du deuxième piston (32b) sur lequel se trouve la boîte ayant ce couvercle est monté jusqu'à la position prescrite d'élévation dans la deuxième colonne (104b),
les moyens (106) de retenue ménageant dans la première position de retenue une largeur de passage inférieure à celle du fond de la boîte de Petri pour la soutenir et supérieure à la largeur du deuxième plateau (320b) du deuxième piston (32b) pour permettre sa descente sous la deuxième colonne (104b).

## Revendications

1. Dispositif de distribution d'un produit prescrit dans au moins une boîte de Petri, chaque boîte (P) de Petri comportant un couvercle (C) amovible apte à être disposé sur un fond (F) moins large que celui-ci,
le dispositif comportant des moyens d'amenée de la boîte de Petri à un poste (40) de distribution, où la boîte de Petri est maintenue avec un espace entre son couvercle et son fond pour permettre la distribution du produit par une tête (6) de distribution du produit comportant un embout (61) de sortie de produit vers le fond lorsque l'embout est dans une position de distribution,
**caractérisé en ce que** le dispositif comporte en outre un piston déterminé (32b) comportant un plateau déterminé (320b) de support du fond (F) au poste (40) de distribution du produit et des moyens d'entraînement en rotation du plateau déterminé (320b) sur lui-même par rapport à la tête (6) de distribution, agencés pour faire tourner le fond (F) par rapport à la tête (6) de distribution se trouvant dans la position de distribution du produit dans le fond, dans laquelle l'embout (61) de sortie de produit est agencé par rapport au plateau déterminé (320b) pour distribuer du produit dans le fond (F) mis en rotation,
les moyens d'amenée comportant un organe (31) de transfert du fond (F) jusqu'à une position d'arrêt déterminée au poste (40) de distribution, l'organe (31) de transfert ayant une ouverture supérieure (311b) de support du couvercle (C) de la boîte de Petri au-dessus d'une ouverture inférieure (312b) de support du fond (F) de la boîte de Petri, ledit plateau déterminé (320b) étant apte à traverser les ouvertures (311b, 312b) pour faire remonter le fond de la boîte de Petri dans le couvercle en traversant leur ouverture respective (311b, 312b) de support, lorsque l'organe (31) de transfert se trouve dans la position d'arrêt déterminée au poste (40) de distribution,
le piston déterminé (32b) muni du plateau déterminé (320b) ayant la fonction de faire tourner le fond (F) de la boîte de Petri pendant la distribution du produit dans le fond (F) dans ladite position de distribution et ayant la fonction de soulever le fond (F) de la boîte de Petri au travers de l'ouverture supérieure (311b) pour rentrer le fond (F) dans le couvercle (C) de la boîte de Petri dans ladite position d'arrêt déterminée.

2. Dispositif de distribution suivant la revendication 1, **caractérisé en ce que** la vitesse de rotation du plateau déterminé (320b) sur lui-même est comprise entre 10 et 100 tours par minute.

3. Dispositif suivant l'une quelconque des revendications précédentes, **caractérisé en ce que** les moyens d'entraînement en rotation sont prévus pour faire tourner le plateau déterminé (320b) sur lui-même autour d'un axe géométrique (AX) de rotation, l'embout (61) comporte une extrémité (610) de sortie de produit située à distance de l'axe géométrique (AX) de rotation sur lui-même du plateau déterminé (320b) mis en rotation dans la position de distribution.

4. Dispositif suivant l'une quelconque des revendications précédentes, **caractérisé en ce que** les moyens d'entraînement en rotation sont prévus pour faire tourner le plateau déterminé (320b) sur lui-même autour d'un axe géométrique (AX) de rotation, l'embout (61) de sortie est agencé pour déverser le produit en une zone du fond (F), laquelle zone est située à distance de l'axe géométrique (AX) de rotation du plateau déterminé (320b) mis en rotation dans la position de distribution.

5. Dispositif suivant l'une quelconque des revendications précédentes, **caractérisé en ce que** le plateau déterminé (320b) comporte une surface supérieure (42) concave de son centre vers sa périphérie pour la réception du fond (F) de la boîte de Petri.

6. Dispositif suivant la revendication 5, **caractérisé en ce que** ladite surface supérieure (42) du plateau déterminé (320b) comporte une partie (44) de contact périphérique avec le fond (F) de la boîte de Petri.

7. Dispositif suivant l'une quelconque des revendications 5 et 6, **caractérisé en ce que** le plateau déterminé (32b) comporte un évidement (43) en son centre sur sa surface supérieure (42).

8. Dispositif suivant l'une quelconque des revendications précédentes, **caractérisé en ce que** la surface supérieure (42) du plateau déterminé (320b) comporte une première partie périphérique (44) de contact avec le fond (F) de la boîte de Petri, laquelle première partie (44) de contact est en un matériau présentant une plus grande adhérence que le matériau d'une deuxième partie (45) de la surface supérieure (42) du plateau déterminé (320b), entourée par cette première partie périphérique (44) de contact.

9. Dispositif suivant la revendication 8, **caractérisé en ce que** la première partie périphérique (44) de contact est un matériau caoutchouteux, tandis que la deuxième partie (45) entourée par cette première partie périphérique (44) de contact est métallique.

10. Dispositif suivant l'une quelconque des revendications précédentes, **caractérisé en ce que** le dispositif comporte
au moins des première et deuxième colonnes (104a, 104b) de guidage vertical de boîtes (P) de Petri, aptes à recevoir respectivement des première et deuxième piles de boîtes de Petri à couvercle disposé sur le fond et aptes à être positionnées respectivement au-dessus d'un premier passage inférieur (115a) de boîte de Petri et au-dessus d'un deuxième passage inférieur (115b) de boîtes de Petri, les premier et deuxième passages (115a, 115b) étant prévus sur une partie fixe (3) par rapport au socle (2),
un système (30) de déplacement d'au moins une boîte de Petri de la première colonne (104a) à la deuxième colonne (104b), comportant :
- l'organe (31) mobile de transfert apte à occuper une autre première position d'arrêt, où les ouvertures (311b, 312b) de support se trouvent sous le premier passage (115a), et ladite deuxième position d'arrêt déterminée au poste (40) de distribution, où les ouvertures (311b, 312b) de support se trouvent sous le deuxième passage (115b),
- la tête (6) de distribution agencée pour distribuer ledit produit au-dessus de l'ouverture inférieure (312b) dans le fond (F) de la boîte de Petri sous l'ouverture supérieure (311b) dans ladite deuxième position d'arrêt déterminée,
- un autre premier piston (32a) comportant un autre premier plateau (320a) situé sous le premier passage (115a) et apte à traverser les ouvertures (311b, 312b) pour faire descendre le couvercle et le fond d'une boîte de Petri de la première colonne (104a) sur leur ouverture respective (311b, 312b) de support lorsque l'organe (31) de transfert se trouve dans la première position d'arrêt associée,
- ledit deuxième piston (32b) déterminé comportant ledit deuxième plateau déterminé (320b) situé sous le deuxième passage inférieur (115b) et apte à traverser les ouvertures (311b, 312b) pour faire remonter le couvercle et le fond de la boîte de Petri de leur ouverture respective (311b, 312b) de support dans la deuxième colonne (104b), lorsque l'organe (31) de transfert se trouve dans la deuxième position d'arrêt associée,
l'organe (31) de transfert étant une navette (31) à déplacement en translation par rapport au socle (2) selon un mouvement direct aller et retour des ouvertures (311b, 312b) de support entre l'une et l'autre des première et deuxième positions (P1, P2) d'arrêt.

11. Dispositif suivant la revendication 10, **caractérisé en ce qu'**une colonne (104b) de guidage vertical de boîtes de Petri est disposée au-dessus du deuxième piston déterminé (32b) pour recevoir la boîte (P) de Petri ayant son fond (F) remonté dans son couvercle (C) par ledit deuxième piston déterminé (32b) au poste (40) de distribution, le deuxième piston déterminé (32b) étant agencé pour déplacer la boîte (P) de Petri ayant son fond (F) remonté dans son couvercle (C) de l'organe (31) de transfert à ladite colonne (104b) de guidage vertical de boîtes de Petri, ladite colonne (104b) de guidage vertical de boîtes de Petri comportant des moyens (106) de retenue de la boîte (P) de Petri ayant son fond (F) remonté dans son couvercle (C) ayant été amenée par le deuxième piston déterminé (32b).

12. Procédé de distribution d'un produit prescrit dans au moins une boite de Petri à l'aide du dispositif de distribution suivant l'une quelconque des revendications précédentes, chaque boîte (P) de Petri comportant un couvercle (C) amovible apte à être disposé sur un fond (F) moins large que celui-ci, procédé dans lequel, on ouvre la boîte de Petri pour maintenir son couvercle (C) à distance de son fond (F), on amène le fond (F) à un poste (40) de distribution, on met la tête (6) de distribution dans une position de distribution pour envoyer du produit dans le fond (F) se trouvant au poste (40) de distribution,
**caractérisé en ce que** l'on positionne le fond (F) de la boîte de Petri sur un piston déterminé (32b) comportant un plateau déterminé (320b) tournant sur lui-même pour faire tourner le fond (F) sur lui-même pendant toute la durée de distribution du produit dans le fond (F) depuis la tête (6) de distribution en position de distribution audit poste (40),
le fond (F) est amené jusqu'à une position d'arrêt déterminée au poste (40) de distribution par un organe (31) de transfert ayant une ouverture supérieure (311b) de support du couvercle (C) de la boîte de Petri au-dessus d'une ouverture inférieure (312b) de support du fond (F) de la boîte de Petri, ledit plateau déterminé (320b) étant apte à traverser les ouvertures (311b, 312b) pour faire remonter le fond de la boîte de Petri dans le couvercle en traversant leur ouverture respective (311b, 312b) de support, lorsque l'organe (31) de transfert se trouve dans la position d'arrêt déterminée au poste (40) de distribution,
le piston déterminé (32b) muni du plateau déterminé (320b) faisant tourner le fond (F) de la boîte de Petri pendant la distribution du produit dans le fond (F) dans ladite position de distribution, puis soulevant le fond (F) de la boîte de Petri au travers de l'ouverture supérieure (311b) pour rentrer le fond (F) dans le couvercle (C) de la boîte de Petri dans ladite position d'arrêt déterminée au poste (40) de distribution.

## Patentansprüche

1. Vorrichtung für die Ausgabe eines vorgeschriebenen Produkts in mindestens eine Petrischale, wobei jede Petrischale (P) einen abnehmbaren Deckel (C) aufweist, der auf einem weniger breiten Boden (F) als diese anordenbar ist,
wobei die Vorrichtung Zuführmittel der Petrischale zu einer Ausgabestelle (40) aufweist, wo die Petrischale mit einem Raum zwischen ihrem Deckel und ihrem Boden gehalten wird, um die Ausgabe des Produkts mit einem Produktausgabekopf (6) zu erlauben, der eine Produktausgangsspitze (61) auf den Boden aufweist, wenn sich die Spitze in einer Ausgabeposition befindet,
**dadurch gekennzeichnet, dass** die Vorrichtung ferner einen bestimmten Kolben (32b) aufweist, der eine bestimmte Stützplatte (320b) des Bodens (F) an der Ausgabestelle (40) des Produkts und Rotationsantriebsmittel der bestimmten Platte (320b) um sich selbst im Verhältnis zum Ausgabekopf (6) aufweist, die ausgebildet sind, um den Boden (F) im Verhältnis zum Ausgabekopf (6) zu drehen, der sich in der Ausgabeposition des Produkts in den Boden befindet, wobei in der Ausgabeposition die Produktausgangsspitze (61) im Verhältnis zur bestimmten Platte (320b) ausgebildet ist, um Produkt in den in Rotation versetzten Boden (F) auszugeben,
wobei die Zufuhrmittel ein Verlagerungsorgan (31) des Bodens (F) in eine bestimmte Stoppposition an der Ausgabestelle (40) aufweisen, wobei das Verlagerungsorgan (31) eine obere Stützöffnung (311b) des Deckels (C) der Petrischale über einer unteren Stützöffnung (312b) des Bodens (F) der Petrischale hat, wobei die bestimmte Platte (320b) imstande ist, die Öffnungen (311b, 312b) zu durchqueren, um den Boden der Petrischale in den Deckel zu heben, bei Durchquerung ihrer jeweiligen Stützöffnung (311b, 312b), wenn sich das Verlagerungsorgan (31) in der bestimmten Stoppposition an der Ausgabestelle (40) befindet,
wobei der bestimmte Kolben (32b), der mit der bestimmten Platte (320b) ausgestattet ist, die Aufgabe hat, den Boden (F) der Petrischale während der Ausgabe des Produkts in den Boden (F) in der Ausgabeposition zu drehen und die Aufgabe hat, den Boden (F) der Petrischale durch die obere Öffnung (311b) zu heben, um den Boden (F) in den Deckel (C) der Petrischale in der bestimmten Stoppposition einzusetzen.

2. Ausgabevorrichtung nach Anspruch 1, **dadurch gekennzeichnet, dass** die Rotationsgeschwindigkeit der bestimmten Platte (320b) um sich selbst zwischen 10 und 100 Umdrehungen pro Minute inklusive beträgt.

3. Vorrichtung nach einem der vorangehenden Ansprüche, **dadurch gekennzeichnet, dass** die Rotationsantriebsmittel vorgesehen sind, um die bestimmte Platte (320b) um sich selbst um eine geometrische Rotationsachse (AX) zu drehen, wobei die Spitze (61) ein Produktausgangsende (610) aufweist, das sich von der geometrischen Rotationsachse (AX) um sich selbst der in der Ausgabeposition in Rotation versetzten bestimmten Platte (320b) beabstandet befindet.

4. Vorrichtung nach einem der vorangehenden Ansprüche, **dadurch gekennzeichnet, dass** die Rotationsantriebsmittel vorgesehen sind, um die bestimmte Platte (320b) um sich selbst um eine geometrische Rotationsachse (AX) zu drehen, wobei die Ausgangsspitze (61) ausgebildet ist, um das Produkt in eine Zone des Bodens (F) auszugeben, wobei sich diese Zone von der geometrischen Rotationsachse (AX) der in der Ausgabeposition in Rotation versetzten bestimmten Platte (320b) beabstandet befindet.

5. Vorrichtung nach einem der vorangehenden Ansprüche, **dadurch gekennzeichnet, dass** die bestimmte Platte (320b) eine von ihrem Zentrum zu ihrem Umfang konkave oberer Fläche (42) für die Aufnahme des Bodens (F) der Petrischale aufweist.

6. Vorrichtung nach Anspruch 5, **dadurch gekennzeichnet, dass** die obere Fläche (42) der bestimmten Platte (320b) einen peripheren Kontaktabschnitt (44) mit dem Boden (F) der Petrischale aufweist.

7. Vorrichtung nach einem der Ansprüche 5 und 6, **dadurch gekennzeichnet, dass** die bestimmte Platte (32b) eine Aussparung (43) in ihrer Mitte auf ihrer oberen Fläche (42) aufweist.

8. Vorrichtung nach einem der vorangehenden Ansprüche, **dadurch gekennzeichnet, dass** die obere Fläche (42) der bestimmten Platte (320b) einen ersten peripheren Kontaktabschnitt (44) mit dem Boden (F) der Petrischale aufweist, wobei der erste Kontaktabschnitt (44) aus einem Material ist, das eine größere Haftfähigkeit als das Material eines zweiten Abschnitts (45) der oberen Fläche (42) der bestimmten Platte (320b) hat, der von diesem ersten peripheren Kontaktabschnitt (44) umgeben ist.

9. Vorrichtung nach Anspruch 8, **dadurch gekennzeichnet, dass** der erste periphere Kontaktabschnitt (44) ein Gummimaterial ist, wogegen der zweite, von diesem ersten peripheren Kontaktabschnitt (44) umgebene Abschnitt (45) metallisch ist.

10. Vorrichtung nach einem der vorangehenden Ansprüche, **dadurch gekennzeichnet, dass** die Vorrichtung aufweist:
mindestens eine erste und zweite vertikale Führungssäule (104a, 104b) von Petrischalen (P), die imstande sind, jeweils einen ersten und zweiten Stapel von Petrischalen mit auf dem Boden angeordnetem Deckel aufzunehmen, die jeweils über einem ersten unteren Petrischalendurchgang (115a) und über einem zweiten unteren Petrischalendurchgang (115b) positionierbar sind, wobei der erste und zweite Durchgang (115a, 115b) auf einem im Verhältnis zum Sockel (2) starren Abschnitt (3) vorgesehen sind,
ein Verlagerungssystem (30) mindestens einer Petrischale von der ersten Säule (104a) zur zweiten Säule (104b), das aufweist:
- das mobile Verlagerungsorgan (31), das imstande ist, eine andere erste Stoppposition einzunehmen, in der sich die Stützöffnungen (311b, 312b) unter dem ersten Durchgang (115a) befinden, und die zweite bestimmte Stoppposition an der Ausgabestelle (40), in der sich die Stützöffnungen (311b, 312b) unter dem zweiten Durchgang (115b) befinden,
- den Ausgabekopf (6), der ausgebildet ist, um das Produkt über der unteren Öffnung (312b) in den Boden (F) der Petrischale unter der oberen Öffnung (311b) in der zweiten bestimmten Stoppposition auszugeben,
- einen anderen ersten Kolben (32a), der eine andere erste Platte (320a) unter dem ersten Durchgang (115a) aufweist und imstande ist, die Öffnungen (311b, 312b) zu durchqueren, um den Deckel und den Boden einer Petrischale der ersten Säule (104a) über ihre jeweilige Stützöffnung (311b, 312b) abzusenken, wenn sich das Verlagerungsorgan (31) in der ersten zugeordneten Stoppposition befindet,
- den zweiten bestimmten Kolben (32b), der die zweite bestimmte Platte (320b) aufweist, die sich unter dem zweiten unteren Durchgang (115b) befindet und imstande ist, die Öffnungen (311b, 312b) zu durchqueren, um den Deckel und den Boden einer Petrischale von ihrer jeweiligen Stützöffnung (311b, 312b) in die zweite Säule (104b) anzuheben, wenn sich das Verlagerungsorgan (31) in der zweiten zugeordneten Stoppposition befindet,
wobei das Verlagerungsorgan (31) ein Shuttle (31) mit verschiebender Verlagerung im Verhältnis zum Sockel (2) in einer direkten Hin- und Rückbewegung der Stützöffnungen (311b, 312b) zwischen der einen und der anderen der ersten und zweiten Stoppposition (P1, P2) ist.

11. Vorrichtung nach Anspruch 10, **dadurch gekennzeichnet, dass** eine vertikale Petrischalen-Führungssäule (104b) über dem zweiten bestimmten Kolben (32b) angeordnet ist, um die Petrischale (P) zu empfangen, deren Boden (F) an der Ausgabestelle (40) vom zweiten bestimmten Kolben (32b) in ihren Deckel (C) angehoben wurde, wobei der zweite bestimmte Kolben (32b) ausgebildet ist, um die Petrischale (P), deren Boden (F) in ihren Deckel (C) angehoben wurde, vom Verlagerungsorgan (31) auf die vertikale Petrischalen-Führungssäule (104b) zu verlagern, wobei die vertikale Petrischalen-Führungssäule (104b) Haltemittel (106) der Petrischale (P), deren Boden (F) in ihren Deckel (C) angehoben wurde, aufweist und die vom zweiten bestimmten Kolben (32b) zugeführt wurde.

12. Ausgabeverfahren eines vorgeschriebenen Produkts in mindestens eine Petrischale mit Hilfe einer Ausgabevorrichtung nach einem der vorangehenden Ansprüche, wobei jede Petrischale (P) einen abnehmbaren Deckel (C) aufweist, der auf einem weniger breiten Boden (F) als diese ablegbar ist, wobei bei diesem Verfahren die Petrischale geöffnet wird, um ihren Deckel (C) von ihrem Boden (F) beabstandet zu halten, der Boden (F) an eine Ausgabestelle (40) geführt wird, der Ausgabekopf (6) in eine Ausgabeposition versetzt wird, um Produkt in den Boden (F) zu verbringen, der sich an der Ausgabestelle (40) befindet,
**dadurch gekennzeichnet, dass** der Boden (F) der Petrischale auf einen bestimmten Kolben (32b) positioniert wird, der eine bestimmte Platte (320b) aufweist, die um sich selbst dreht, um den Boden (F) während der gesamten Dauer der Ausgabe des Produkts in den Boden (F) vom Ausgabekopf (6) in Ausgabeposition an der Stelle (40) um sich selbst zu drehen,
der Boden (F) von einem Verlagerungsorgan (31), das eine obere Stützöffnung (311b) des Deckels (C) der Petrischale über eine untere Stützöffnung (312b) des Bodens (F) der Petrischale hat, zu einer bestimmten Stoppposition an der Ausgabestelle (40) geführt wird, wobei die bestimmte Platte (320b) imstande ist, die Öffnungen (311b, 312b) zu durchqueren, um den Boden der Petrischale bei Durchquerung ihrer jeweiligen Stützöffnung (311b, 312b) in den Deckel zu heben, wenn sich das Verlagerungsorgan (31) in der bestimmten Stoppposition an der Ausgabestelle (40) befindet,
wobei der bestimmte Kolben (32b), der mit einer bestimmten Platte (320b) ausgestattet ist, den Boden (F) der Petrischale während der Ausgabe des Produkts in den Boden (F) in der Ausgabeposition dreht, danach den Boden (F) der Petrischale durch die obere Öffnung (311b) hebt, um den Boden (F) in den Deckel (C) der Petrischale in der bestimmten Stoppposition an der Ausgabestelle (40) einzusetzen.

## Claims

1. A device for dispensing a prescribed product into at least one Petri dish, each Petri dish (P) comprising a removable lid (C) able to be placed over a bottom part (F) of smaller width,
the device comprising transfer means for transferring the Petri dish to a product dispensing station (40) where the Petri dish is held with a space between its lid and bottom part to allow the dispensing of product via a product dispensing head (6) comprising an outlet nozzle (61) dispensing product into the bottom part when the outlet nozzle is in a product dispensing position,
**characterized in that** the device further comprises a determined piston (32b) comprising a determined tray (320b) supporting the bottom part (F) at the product dispensing station (40), and means for driving the determined tray (320b) in rotation around itself relative to the product dispensing head (6), arranged to cause the bottom part (F) to rotate relative to the product dispensing head (6) lying in the product dispensing position into the bottom part, wherein the outlet nozzle (61) is arranged relative to the determined tray (320b) to dispense product into the bottom part (F) set in rotation,
the transfer means comprising a transfer member (31) to transfer the bottom part (F) as far as a determined stop position at the product dispensing station (40), the transfer member (31) having an upper supporting opening (311b) for supporting the lid (C) of the Petri dish above a lower supporting opening (312b) for supporting the bottom part (F) of the Petri dish, said determined tray (320b) being capable of passing through the openings (311b, 312b) to lift the bottom part of the Petri dish into the lid by passing through their respective supporting opening (311b, 312b), when the transfer member (31) lies in the determined stop position at the product dispensing station (40),
the determined piston (32b) provided with the determined tray (320b) having the function of rotating the bottom part (F) of the Petri dish during the dispensing of product into the bottom part(F) in said product dispensing position, and having the function of lifting the bottom part (F) of the Petri dish through the upper supporting opening (311b) to insert the bottom part (F) into the lid (C) of the Petri dish in said determined stop position.

2. The dispensing device according to claim 1, **characterized in that** a speed of rotation of the determined tray (320b) around itself is between 10 and 100 rotations per minute.

3. The device according to any one of the preceding claims, **characterized in that** the means for driving in rotation are provided to cause the determined tray (320b) to rotate around itself about a geometric axis of rotation (AX), the outlet nozzle (61) comprises a product outlet end (610) located away from the geometric axis of rotation (AX) of the determined tray (320b) set in rotation around itself in the product dispensing position.

4. The device according to any one of the preceding claims, **characterized in that** the means for driving in rotation are designed to cause the determined tray (320b) to rotate around itself about a geometric axis (AX) of rotation, the outlet nozzle (61) is arranged to dispense product into a region of the bottom part (F), this region lying at a distance from the geometric axis (AX) of rotation of the determined tray (320b) set in rotation in the product dispensing position.

5. The device according to any one of the preceding claims, **characterized in that** the determined tray (320b) comprises an upper surface (42) that is concave from its centre towards its periphery to receive the bottom part (F) of the Petri dish.

6. The device according to claim 5, **characterized in that** said upper surface (42) of the determined tray (320b) comprises a peripheral contacting part (44) for contacting with the bottom part(F) of the Petri dish.

7. The device according to either of claims 5 and 6, **characterized in that** the determined tray (32b) carries a recess (43) in its centre on its upper surface (42).

8. The device according to any one of the preceding claims, **characterized in that** the upper surface (42) of the determined tray (320b) comprises a first peripheral contacting part (44) for contacting with the bottom part(F) of the Petri dish, wherein this first peripheral contacting part (44) is in a material having greater adherence than the material of a second part (45) of the upper surface (42) of the determined tray (320b), surrounded by this first peripheral contacting part (44).

9. The device according to claim 8, **characterized in that** the first peripheral contacting part (44) is in rubber material, whereas the second part (45) surrounded by this first peripheral contacting part (44) is metallic.

10. The device according to any one of the preceding claims, **characterized in that** the device comprises:
at least first and second columns (104a, 104b) for the vertical guiding of Petri dishes (P), capable of receiving first and second stacks of Petri dishes respectively with lid placed over the bottom part and capable of being respectively positioned above a first passageway (115a) for passage of Petri dishes and above a second passageway (115b) for passage of Petri dishes, the first and second passageways (115a, 115b) being provided on a fixed part (3) relative to a base (2),
a system (30) for moving at least one Petri dish from the first column (104a) to the second column (104b) comprising:
- the transfer member (31) being mobile and capable of taking up another first stop position in which the supporting openings (311b, 312b) lie under the first passageway (115a), and said second determined stop position at the product dispensing station (40) in which the supporting openings (311b, 312b) lie under the second passageway (115b),
- the product dispensing head (6) arranged to dispense said product above the lower supporting opening (312b) into the bottom part (F) of the Petri dish under the upper supporting opening (311b) in said second determined stop position,
- another first piston (32a) comprising another first tray (320a) located under the first passageway (115a) and capable of passing through the supporting openings (311b, 312b) to lower the lid and the bottom part of a Petri dish of the first column (104a) onto their respective supporting opening (311b, 312b) when the transfer member (31) lies in the associated first stop position,
- said second determined piston (32b) comprising said second determined tray (320b) located under the second lower passageway (115b) and capable of passing through the supporting openings (311b, 312b) to lift the lid and the bottom part of the Petri dish from their respective supporting opening (311b, 312b) into the second column (104b) when the transfer member (31) lies in the associated second stop position,
the transfer member (31) being a shuttle (31) with translational movement relative to the base (2) in direct back and forth movement of the supporting openings (311b, 312b) between each of the first and second stop positions (P1, P2),

11. The device according to claim 10, **characterized in that** one of the columns (104b) for the vertical guiding of Petri dishes is arranged above the second determined piston (32b) to receive the Petri dish (P) having its bottom part (F) lifted into its lid (C) via said second determined piston (32b) at the product dispensing station (40), the second determined piston (32b) being arranged to move the Petri dish (P) with its bottom part (F) lifted into its lid (C) from the transfer member (31) to said column (104b) for vertical guiding of the Petri dishes, said column (104b) for vertical guiding of the Petri dishes comprising means (106) for retaining the Petri dish (P) having its bottom part (F) lifted into its lid (C) which has been brought by the second determined piston (32b).

12. A method for dispensing a prescribed product into at least one Petri dish using the dispensing device according to any one of the preceding claims, each Petri dish (P) comprising a removable lid (C) able to be placed over a bottom part (F) of smaller width, a method in which the Petri dish is opened to hold its lid (C) away from its bottom part (F), the bottom part (F) is brought to a product dispensing station (40), the dispensing head (6) is placed in a product dispensing position to dispense product into the bottom part (F) located at the dispensing station (40),
**characterized in that** the bottom part (F) of the Petri dish is positioned on a determined piston (32b) comprising a determined tray (320b) rotating around itself, to cause the bottom part (F) to rotate around itself throughout the entire time that product is dispensed into the bottom part (F) by the product dispensing head (6) in product dispensing position at said product dispensing station (40),
the bottom part(F) is brought as far as a determined stop position at the product dispensing station (40) by a transfer member (31) having an upper supporting opening (311b) for supporting the lid (C) of the Petri dish above a lower supporting opening (312b) for supporting the bottom part(F) of the Petri dish, said determined tray (320b) being capable of passing through the supporting openings (311b, 312b) to lift the bottom of the Petri dish into the lid by passing through their respective supporting opening (311b, 312b), when the transfer member (31) lies in the determined stop position at the product dispensing station (40),
wherein the determined piston (32b) provided with the determined tray (320b) rotates the bottom part(F) of the Petri dish while product is dispensed into the bottom part (F) in said product dispensing position, then lifts the bottom part (F) of the Petri dish through the upper supporting opening (311b) to insert the bottom part (F) into the lid (C) of the Petri dish in said determined stop position at the product dispensing station (40).
